# EUROPÄISCHE PATENTSCHRIFT

(11) **EP 2 261 336 B1**
(45) Veröffentlichungstag und Bekanntmachung des Hinweises auf die Patenterteilung: **16.10.2013**
(21) Anmeldenummer: 10009677.5
(22) Anmeldetag: 01.12.2004
(51) Int. Cl.: C12N 15/29, C07K 14/415, A61K 38/56, A61K 39/36, A61K 31/7088

(54) **DNA-Sequenz und rekombinante Herstellung von Gruppe-4 Majorallergenen aus Getreiden**
DNA-SEQUENCE AND RECOMBINANT PRODUCTION OF GROUP 4 MAJOR ALLERGENS FROM CEREALS
SEQUENCE D'ADN ET PRODUCTION RECOMBINANTE D'ALLERGENES MAJEURS DU GROUPE 4 A PARTIR DE CEREALES

(30) Priorität: 16.12.2003 DE 10359351
(43) Veröffentlichungstag der Anmeldung: 15.12.2010
(62) Teilanmeldung aus: 04803421.9
(73) Patentinhaber: Merck Patent GmbH, 64293 Darmstadt (DE)
(72) Erfinder: Fiebig, Helmut, Prof., 21493 Schwarzenbek (DE); Nandy, Andreas, Dr., 22175 Hamburg (DE); Cromwell, Oliver, Dr., 23701 Suesel-Fassendorf (DE)

(56) Entgegenhaltungen:
- WO-A-03/025009
- WO-A-2004/000881
- STUMVOLL S ET AL: "Purification, structural and immunological characterization of a timothy grass (Phleum pratense) pollen allergen, Phl p 4, with cross-reactive potential" BIOLOGICAL CHEMISTRY, Bd. 383, Nr. 9, September 2002 (2002-09), Seiten 1383-1396, XP002260346 ISSN: 1431-6730
- ASTWOOD J D ET AL: "Cloning and expression pattern of Hor v 9, the group 9 pollen isoallergen from barley" GENE: AN INTERNATIONAL JOURNAL ON GENES AND GENOMES, ELSEVIER SCIENCE PUBLISHERS, BARKING, GB, Bd. 182, Nr. 1-2, 5. Dezember 1996 (1996-12-05), Seiten 53-62, XP004071930 ISSN: 0378-1119
- SCHEINER O ET AL: "CDNA CLONING OF THE MAJOR POLLEN ALLERGENS FROM TIMOTHY GRASS (PHLEUM PRATENSE) AND RYE (SECALE CEREALE). DIAGNOSIS OF GRASS POLLEN ALLERGY WITH RECOMBINANT ALLERGENS" MOLECULAR BIOLOGY AND IMMUNOLOGY OF ALLERGENS, 1993, Seiten 153-156, XP009040110
- RIHS H P ET AL: "POLYMERASE CHAIN REACTION BASED CDNA CLONING OF WHEAT PROFILIN: A POTENTIAL PLANT ALLERGEN" INTERNATIONAL ARCHIVES OF ALLERGY AND IMMUNOLOGY, Bd. 105, Januar 1994 (1994-01), Seiten 190-194, XP000604627 ISSN: 1018-2438
- FAHLBUSCH B ET AL: "Detection and quantification of group 4 allergens in grass pollen extracts using monoclonal antibodies" CLINICAL AND EXPERIMENTAL ALLERGY, BLACKWELL SCIENTIFIC PUBLICATIONS, LONDON, GB, Bd. 28, Nr. 7, Juli 1998 (1998-07), Seiten 799-807, XP002260345 ISSN: 0954-7894
- DATABASE EMBL 19. November 2004 (2004-11-19), XP002332685 Database accession no. AJ862834

## Beschreibung

### Hintergrund der Erfindung

Die vorliegende Erfindung betrifft die Bereitstellung von DNA-Sequenzen von Gruppe-4 Majorallergenen aus Getreiden (*Triticeae*). Die Erfindung schließt auch Fragmente, Neukombinationen von Teilsequenzen und Punktmutanten mit hypoallergener Wirkung ein. Die rekombinanten DNA-Moleküle und die abgeleiteten Polypeptide, Fragmente, Neukombinationen von Teilsequenzen und Varianten können zur Therapie von pollenallergischen Krankheiten genutzt werden. Die rekombinant hergestellten Proteine können zur *In-vitro-* und *In-vivo*-Diagnostik von Pollenallergien eingesetzt werden.

Allergien vom Typ 1 haben weltweite Bedeutung. Bis zu 20 % der Bevölkerung in industrialisierten Ländern leiden unter Beschwerden wie allergischer Rhinitis, Konjunktivitis oder Bronchialasthma. Diese Allergien werden durch in der Luft befindliche Allergene (Aeroallergene), die von Quellen unterschiedlicher Herkunft wie Pflanzenpollen, Milben, Katzen oder Hunden freigesetzt werden, hervorgerufen. Bis zu 40 % dieser Typ 1-Allergiker wiederum zeigen spezifische IgE-Reaktivität mit Gräserpollenallergenen, unter anderem Getreidepollenallergenen (Freidhoff et al., 1986, J. Allergy Clin. Immunol. 78, 1190-2001). Eine besondere Bedeutung unter den Getreidepollenallergenen besitzen die Allergene von Roggen.

Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Molekülen. Werden zwei IgE-Moleküle durch ein Allergen miteinander vernetzt, führt dies zur Ausschüttung von Mediatoren (z. B. Histamin, Prostaglandine) und Zytokinen durch die Effektorzelle und damit zu den entsprechenden klinischen Symptomen.

In Abhängigkeit von der relativen Häufigkeit mit der die einzelnen Allergenmoleküle mit den IgE-Antikörpern von Allergikern reagieren, wird zwischen Major- und Minorallergenen unterschieden.

Die Allergene aus den Pollen von verschiedenen Spezies aus der Familie er Gräser (*Poaceae*) werden in Grupppen eingeteilt, die untereinander homolog sind.

Insbesondere die Moleküle der Majorallergengruppe 4 weisen untereinander eine hohe immunologische Kreuzreaktivität sowohl mit monoklonalen Mausantikörpern als auch mit humanen IgE-Antikörpern auf (Fahlbusch et al., 1993 Clin. Exp. Allergy 23:51-60; Leduc-Brodard et al., 1996, J. Allergy Clin. Immunol. 98:1065-1072; Su et al., 1996, J. Allergy Clin. Immunol. 97:210; Fahlbusch et al., 1998, Clin. Exp. Allergy 28:799-807; Gavrovic-Jankulovic et al., 2000, Invest. Allergol. Clin. Immunol. 10 (6):361-367; Stumvoll et al. 2002, Biol. Chem. 383:1383-1396; Grote et al., 2002, Biol. Chem. 383:1441-1445; Andersson und Lidholm, 2003, Int. Arch. Allergy Immunol. 130:87-107; Mari, 2003, Clin. Exp. Allergy, 33 (1):43-51).

Von keinem der Gruppe-4-Majorallergene ist bisher eine vollständige DNA-Sequenz bekannt.

Von dem Gruppe-4 Allergen aus *Dactylus glomerata* sind bisher lediglich Peptide durch enzymatischen Abbau gewonnen und sequenziert worden:
DIYNYMEPYVSK (SEQ ID NO 13),
VDPTDYFGNEQ (SEQ ID NO 14),
ARTAWVDSGAQLGELSY (SEQ ID NO 15)
und GVLFNIQYVNYWFAP (SEQ ID NO 16, Leduc-Brodard et al., 1996, J. Allergy Clin. Immunol. 98: 1065-1072).
Auch vom Gruppe-4 Allergen des subtropischen Bermuda-Grases (*Cynodon dactylon*) sind durch Proteolyse Peptide erhalten und sequenziert worden:
KTVKPLYIITP (SEQ ID NO 17),
KQVERDFLTSLTKDIPQLYLKS (SEQ ID NO 18),
TVKPLYIITPITAAMI (SEQ ID NO 19),
LRKYGTAADNVIDAKVVDAQGRLL (SEQ ID NO 20),
KWQTVAPALPDPNM (SEQ ID NO 21),
VTWIESVPYIPMGDK (SEQ ID NO 22),
GTVRDLLXRTSNIKAFGKY (SEQ ID NO 23),
TSNIKAFGKYKSDYVLEPIPKKS (SEQ ID NO 24),
YRDLDLGVNQVVG (SEQ ID NO 25),
SATPPTHRSGVLFNI (SEQ ID NO 26),
und AAAALPTQVTRDIYAFMTPYVSKNPRQAYVNYRDLD (SEQ ID NO 27,
Liaw et al., 2001, Biochem. Biophys. Research Communication 280: 738-743 ).

Für *Lolium perenne* wurden für das basische Gruppe-4 Allergen Peptidfragmente mit den folgenden Sequenzen beschrieben: FLEPVLGLIFPAGV (SEQ ID NO 28) und GLIEFPAGV (SEQ ID NO 29, Jaggi et al., 1989, Int. Arch. Allergy Appl. Immunol. 89: 342-348).

Als erste Sequenz eines Allergens der Gruppe 4 wurde von den Erfindern der vorliegenden Patentanmeldung die noch unveröffentlichte Sequenz des Phl p 4 aus *Phleum pratense* aufgeklärt (SEQ ID NO 11) und in der internationalen Anmeldung WO 04/000881 beschrieben.

Über die Sequenzen der Gruppe-4-Majorallergene aus Getreiden (*Triceae*) ist bisher nichts bekannt.

Die der vorliegenden Erfindung zugrunde liegende Aufgabe bestand daher in der Bereitstellung von DNA-Sequenzen von Gruppe-4 Majorallergenen aus Getreiden, insbesondere des Allergens Sec c 4 aus Roggen (*Secale cerale*) (SEQ ID NO 1, 3), Hor v 4 aus Gerste (*Hordeum vulgare*) (SEQ ID NO 5) und Tri a 4 aus Weizen (*Triticum aestivum*) (SEQ ID NO 7, 9) sowie von entsprechenden rekombinanten DNA-Molekülen, auf deren Grundlage die Allergene als Protein exprimiert und einer pharmakologisch bedeutsamen Verwertung als solches oder in veränderter Form zugänglich gemacht werden kann. Die Sequenz des Phl p 4 (SEQ ID NO 11) war Ausgangspunkt für die vorliegende Erfindung.

### Verzeichnis der erfindungsgemäßen Sequenzen

Den DNA- und Protein-Sequenzen der reifen Allergene gemäß SEQ ID NO 1-10 geht eine Signalsequenz voraus. Mit den TGA oder TAG Stopcodons in den DNA-Sequenzen endet der kodierende Bereich.
- DNA-Sequenz des Sec c 4. (a) Isoform Sec c 4.01 (SEQ ID NO 1), (b) Isoform Sec c 4.02 (SEQ ID NO 3).
- Von den DNA-Sequenzen gemäß SEQ ID NO 1 und 3 abgeleitete Protein-Sequenzen (SEQ ID NO 2, 4).
- DNA-Sequenz des Hor v 4 (SEQ ID NO 5).
- Von der DNA-Sequenz gemäß SEQ ID NO 5 abgeleitete Protein-Sequenz (SEQ ID NO 6).
- DNA-Sequenz des Tri a 4. (a) Isoform Tri a 4.01 (SEQ ID NO 7), (b) Isoform Tri a 4.02 (SEQ ID NO 9).
- Von den DNA-Sequenzen gemäß SEQ ID NO 7 und 9 abgeleitete Protein-Sequenzen (SEQ ID NO 8, 10).
- DNA-Sequenz des Phl p 4 (SEQ ID NO 11), gemäß SEQ ID NO 5 aus der WO 04/000881.
- Proteinsequenz des Phl p 4 (SEQ ID NO 12), gemäß SEQ ID NO 6 aus der WO 04/000881.

### Beschreibung der Erfindung

Mit der vorliegenden Erfindung werden nun erstmals DNA-Sequenzen der Getreidepollenhauptallergene Sec c 4, Hor v 4 und Tri a 4, gemäß SEQ ID NO 1, 3, 5, 7, und 9, bereit gestellt.
Gegenstand der vorliegenden Erfindung ist daher ein DNA-Molekül mit der Nukleotidsequenz gemäß SEQ ID NO 5.

Die Erfindung betrifft weiterhin zu den erfindungsgemäßen DNA-Sequenzen homologe Sequenzen bzw. entsprechende DNA-Moleküle von Gruppe-4-Allergenen aus anderen *Poaceae* wie beispielsweise *Lolium perenne, Dactylis glomerata, Poa pratensis, Cynodon dactylon* und *Holcus lanatus,* die aufgrund der bestehenden Sequenzhomologie mit den erfindungsgemäßen DNA-Sequenzen unter stringenten Bedingungen hybridisieren, bzw. bezüglich der erfindungsgemäßen Allergene eine immunologische Kreuzraktivität aufweisen.

Die Erfindung schließt dabei auch Fragmente, Neukombinationen von Teilsequenzen und Punktmutanten mit hypoallergener Wirkung ein.

Gegenstand der Erfindung sind daher weiterhin entsprechende Teilsequenzen, einer Kombination von Teilsequenzen bzw. Austausch-, Eliminierungs- oder Additionsmutanten, welche für ein immunmodulatorisches, T-Zell-reaktives Fragment eines Gruppe-4-Allergens der Poaceae kodieren. Mit der Kenntnis der DNA-Sequenz der natürlich vorkommenden Allergene ist es nun möglich, diese Allergene als rekombinante Proteine herzustellen, die in der Diagnostik und Therapie von allergischen Erkrankungen Verwendung finden können (Scheiner and Kraft, 1995, Allergy 50: 384-391).

Ein klassischer Ansatz zur wirksamen therapeutischen Behandlung von Allergien stellt die Spezifische Immuntherapie oder Hyposensibilisierung dar (Fiebig, 1995, Allergo J. 4 (6): 336-339, Bousquet et al., 1998, J. Allergy Clin. Immunol. 102(4): 558-562). Dabei werden dem Patienten natürliche Allergenextrakte in steigenden Dosen subkutan injiziert. Allerdings besteht bei dieser Methode die Gefahr von allergischen Reaktionen oder sogar eines anaphylaktischen Schocks. Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen (Fiebig, 1995, Allergo J. 4 (7): 377-382). Eine noch weitergehende Therapieoptimierung wäre mit rekombinant hergestellten Allergenen möglich. Definierte, ggfs. auf die individuellen Sensibilisierungsmuster der Patienten abgestimmte Cocktails von hochreinen, rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten.
Realistische Perspektiven, die zu einer sicheren Hyposensibilisierung mit Expressionsprodukten führen können, bieten gezielt mutierte rekombinante Allergene, bei denen IgE-Epitope spezifisch deletiert werden, ohne die für die Therapie essentiellen T-Zell Epitope zu beeinträchtigen (Schramm et al., 1999, J. Immunol. 162: 2406-2414).

Eine weitere Möglichkeit zur therapeutischen Beeinflussung des gestörten TH-Zell-Gleichgewichtes bei Allergikern ist die immuntherapeutische DNA-Vakzinierung. Dabei handelt es sich um eine Behandlung mit expressionsfähiger DNA, die für die relevanten Allergene kodiert. Erste experimentelle Belege für die allergenspezifische Beeinflussung der Immunantwort konnte an Nagern durch Injektion von Allergen-kodierender DNA erbracht werden (Hsu et al., 1996, Nature Medicine 2 (5): 540-544).

Gegenstand der vorliegenden Erfindung ist daher auch ein vor- oder nachstehend beschriebenes DNA-Molekül bzw. ein entsprechender rekombinanter Expressionsvektor als Arzneimittel.

Die entsprechenden rekombinant hergestellten Proteine können zur Therapie sowie zur *in vitro-* und *in vivo*-Diagnostik von Pollenallergien eingesetzt werden.
Zur Herstellung des rekombinanten Allergens wird die klonierte Nukleinsäure in einen Expressionsvektor ligiert und dieses Konstrukt in einem geeigneten Wirtsorganismus exprimiert. Nach biochemischer Reinigung steht dieses rekombinante Allergen zur Detektion von IgE-Antikörpern in etablierten Verfahren zur Verfügung.

Gegenstand der vorliegenden Erfindung ist daher weiterhin ein rekombinanter Expressionsvektor, enthaltend ein vor- oder nachstehend beschriebenes DNA-Molekül, funktionell verbunden mit einer Expressionskontrollsequenz und ein Wirtsorganismus, transformiert mit besagtem DNA-Molekül oder besagtem Expressionsvektor.

Ebenfalls erfindungsgegenständlich ist die Verwendung mindestens eines zuvor beschriebenen DNA-Moleküls oder mindestens eines zuvor beschriebenen Expressionsvektors zur Herstellung eines Arzneimittels zur immuntherapeutischen DNA-Vakzinierung von Patienten mit Allergien, an deren Auslösung Gruppe-4-Allergene der Poaceae, vorzugsweise *Triticeae*, insbesondere Sec c 4, Hor v 4, Tri a 4, beteiligt sind und/oder zur Prävention solcher Allergien.

Wie bereits ausgeführt kann die Erfindung als eine essentielle Komponente in einem rekombinanten allergen- oder nukleinsäurehaltigen Präparat zur spezifischen Immuntherapie angewendet werden. Hierbei bieten sich mehrere Möglichkeiten. Zum einen kann das in der Primärstruktur unveränderte Protein Bestandteil des Präparates sein. Zum anderen kann durch gezielte Deletion von IgE-Epitopen des Gesamtmoleküls oder der Herstellung von einzelnen Fragmenten, die für T-Zell Epitope kodieren, erfindungsgemäß eine hypoallergene (allergoide) Form zur Therapie verwendet werden, um unerwünschte Nebenwirkungen zu vermeiden. Schließlich wird durch die Nukleinsäure an sich, wenn sie mit einem eukaryontischen Expressionsvektor ligiert wird, ein Präparat geschaffen, das direkt appliziert den allergischen Immunzustand im therapeutischen Sinne verändert.

Desweiteren handelt es sich bei der vorliegenden Erfindung um die von einem oder mehreren der zuvor beschriebenen DNA-Moleküle kodierten Polypeptide, vorzugsweise in ihrer Eigenschaft als Arzneimittel.
Dabei handelt es sich um das Protein entsprechend einer Aminosäuresequenz gemäß SEQ ID NO 6. Insbesondere handelt es sich um das reife Protein (ohne Signalsequenzanteil), beginnend mit der Aminosäure 23 (SEQ ID NO 6). Weiterhin betrifft die Erfindung Proteine, welche diese Aminosäuresequenzen oder Teile dieser Sequenzen enthalten,

Die Erfindung betrifft demgemäß auch ein Verfahren zur Herstellung solcher Polypeptide durch Kultivieren eines Wirtsorganismus und Gewinnung des entsprechenden Polypeptids aus der Kultur.

Ebenfalls erfindungsgegenständlich ist die Verwendung mindestens eines zuvor beschriebenen Polypeptides zur Herstellung eines Arzneimittels zur Diagnose und/oder Behandlung von Allergien, an deren Auslösung Gruppe-4-Allergene der *Poaceae*, vorzugsweise *Triticeae,* insbesondere

Sec c 4, Hor v 4, Tri a 4, beteiligt sind sowie zur Prävention solcher Allergien.

Bei der Ermittlung der erfindungsgemäßen Protein- und DNA-Sequenzen wurde wie folgt vorgegangen:

### Sec c 4 aus Roggen

1. Ausgehend von der DNA-Sequenz des Phl p 4 (SEQ ID NO 12, WO 04/000881) wurden spezifische Primer (Tab. 1) generiert, die von der Phl p 4 Sequenz abgeleitet wurden. Durch PCR mit den Primern #87 und #83 konnten fünf Klone aus Roggenpollen DNA gewonnen werden. Das diesen Klonen entsprechende, amplifizierte Sec c 4-Genfragment-1 kodiert für ein den Aminosäuren 68-401 des Phl p 4 (SEQ ID NO 12) entsprechendes Polypeptid.
2. Mit der partiellen Sec c 4-Sequenz wurde eine EST-Datenbankrecherche durchgeführt. Es konnten jedoch keine homologen Sequenzen in auf Roggen spezialisierte EST-Datenbanken gefunden werden. Statt dessen wurden einzelne, homologe, nicht überlappende EST-Fragmente in auf Gerste und Weizen spezialisierten EST-Datenbanken gefunden. Einzelne EST-Fragmente reichen in den 5'-UTR, andere in den 3'-UTR Bereich (UTR = nicht-translatierter Bereich) der entsprechenden Gene hinein.
3. Aus den in den Datenbanken gefundenen EST-Sequenzen lässt sich jedoch kein komplettes Gruppe-4-Gen aus Weizen oder Gerste konstruieren, da diese Sequenzen nicht überlappen und kein homologes Gruppe-4-Gen bekannt ist. Anhand der Phl p 4-Sequenz (SEQ ID NO 11) und des in Schritt 1 erhaltenen Sec c 4-Fragmentes konnten diese EST-Sequenzen jedoch zugeordnet werden und dienten als Vorlage für die Herstellung von PCR-Primern.
4. Mit Hilfe der so hergestellten Primer #195 und #189 konnten drei Klone durch PCR erhalten werden. Der Primer #195 wurde aus einer Gerste-EST-Sequenz abgeleitet, der Primer #189 ist ein Phl p 4-spezifischer Primer und überlappt das Phl p 4-Stoppcodon sowie die Codons der 10 C-terminalen Phl p 4-Aminosäuren. Das so amplifizierte Sec c 4-Genfragment-2 kodiert für ein Polypeptid, beginnend innerhalb der Signalsequenz und endend mit der Position, die der Position 490 des Phl p 4 entspricht. Dieses Polypeptid deckt den N-Terminus von Sec c 4 ab.
5a. Drei weitere Klone wurden durch PCR mit den Primern #195 und #202 erhalten. Beide Primer wurden aus Gerste EST-Sequenzen abgeleitet. Das amplifizierte Sec c 4-Gen-3 kodiert für die korrespondierenden Aminosäuren beginnend innerhalb der Signalsequenz und endend am C-Terminus von Sec c 4.
   Die komplette Sequenz des reifen Sec c 4 ist somit in der bestimmten Sequenz enthalten.

Die beiden nächsten Schritte 5b und 5c dienen der Absicherung des im Schritt 5a erhaltenen Ergebnisses:
5b. Ein weiterer Klon wurde durch PCR mit den Primern #195 und #203 erhalten. Primer #195 wurde von einer Gerste EST-Sequenz abgeleitet, Primer #203 von einer Weizen EST Sequenz. Das amplifizierte Sec c 4 Gen kodiert für die korrespondierenden Aminosäuren beginnend innerhalb der Signalsequenz und endend am C-Terminus von Sec c 4. Die komplette Sequenz des reifen Sec c 4 ist daher in der bestimmten Sequenz enthalten.
5c. Ein weiterer Klon wurde durch PCR mit den Primern #195 und #198 erhalten. Auch Primer #198 Das amplifizierte Sec c 4 Gen kodiert für die korrespondierenden Aminosäuren beginnend innerhalb der Signalsequenz und endend am C-Terminus von Sec c 4. Die komplette Sequenz des reifen Sec c 4 ist daher in der bestimmten Sequenz enthalten.

Es wurden zwei Isoformen Sec c 4.01 und 4.02 aufgefunden. Die reifen Allergene beginnen mit der Aminosäure 23 der Sequenzen gemäß SEQ ID NO 2, 4, und 6.

### Hor v 4 aus Gerste

Mit Hilfe der wie zuvor beschrieben erhaltenen Sec c 4-Sequenzen konnten in EST-Datenbanken von *Hordeum vulgare* homologe EST-Fragmente gefunden wurde. Diese Fragmente überlappen jedoch nicht zu einem kompletten Gen. Anhand der gefundenen EST-Sequenzen konnten jedoch Hor v 4-spezifische Primer generiert werden, die für eine Amplifikation des Hor v 4-Gens aus genomischer DNA verwendet wurden.

Insgesamt wurden 15 Klone analysiert.
4 Klone wurden durch PCR mit den Primern #195 und #198 erhalten.
4 Klone wurden durch PCR mit den Primern #195 und #202 erhalten.
3 Klone wurden durch PCR mit den Primem #194 und #198 erhalten.
4 Klone wurden durch PCR mit den Primern #194 und #202 erhalten.

Die abgeleitete Proteinsequenz beginnt innerhalb der Signalsequenz von Hor v 4 und reicht bis zum C-terminalen Ende des Proteins (ab Aminosäure 23 von SEQ ID NO 6).

### Tri a 4 aus Weizen

Mit Hilfe der wie zuvor beschrieben erhaltenen Sec c 4-Sequenz konnten in EST-Datenbanken von *Triticum aestivum* homologe EST-Fragmente gefunden wurde. Diese Fragmente überlappen jedoch nicht zu einem kompletten Gen. Anhand der gefundenen EST-Sequenzen konnten jedoch die Tri a 4-spezifische Primer #199, #203, #204 und #206 generiert werden, die für eine Amplifikation des Tri a 4 Gens aus genomischer DNA verwendet wurden.

Insgesamt wurden 13 Klone analysiert.
4 Klone wurden durch PCR mit den Primern #204 und #203 erhalten.
4 Klone wurden durch PCR mit den Primern #204 und #199 erhalten.
3 Klone wurden durch PCR mit den Primern #206 und #203 erhalten.
4 Klone wurden durch PCR mit den Primern #206 und #199 erhalten.

Die abgeleiteten Proteinsequenzen beginnen innerhalb der Signalsequenz von Tri a 4 und reichen bis zum C-terminalen Ende des Proteins.
Es wurden zwei Varianten Tri a 4.01 (ab Aminosäure 22 von SEQ ID NO 8) und Tri a 4.02 (ab Aminosäure 22 von SEQ ID NO 10) aufgefunden.

Zur Herstellung der rekombinanten erfindungsgemäßen Allergene wurden die DNA-Sequenzen gemäß SEQ ID NO 1, 3, 5, 7 und 9 in Expressionsvektoren (z.B. pProEx, pSE 380) eingebaut. Für die aus der Proteinsequenzierung bekannten N-terminalen Aminosäuren wurden *E*. *coli* optimierte Codons verwendet.

Nach der Transformation in *E*. *coli*, der Expression und der Reinigung des rekombinanten erfindungsgemäßen Allergene durch verschiedene Trenntechniken wurde die erhaltenen Proteine einem Refoldingprozess unterworfen.
Beide Allergene können zur hochspezifischen Diagnostik von Graspollenallergien eingesetzt werden. Diese Diagnostik kann *in vitro* durch die Detektion von spezifischen Antikörpern (IgE, IgG1 - 4, IgA) und die Reaktion mit IgE-beladenen Effektorzellen (z. B. Basophile aus dem Blut) oder *in vivo* durch Hauttest-Reaktionen und Provokation am Reaktionsorgan erfolgen. Die Reaktion der erfindungsgemäßen Allergene mit T-Lymphozyten von Graspollenallergikern können durch die allergenspezifische Stimulierung der T-Lymphozyten zur Proliferation und Zytokinsynthese sowohl mit T-Zellen in frisch präparierten Blutlymphozyten als auch an etablierten nSec c 4, nHor v 4 bzw. nTri a 4-reaktiven T-Zell-Linien und -Klonen nachgewiesen werden.

Durch ortsgerichte Mutagenese wurden die für die Cysteine kodierenden Tripletts so verändert, dass sie für andere Aminosäuren, bevorzugt Serin, kodieren. Es wurden sowohl Varianten hergestellt, bei denen einzelne Cysteine ausgetauscht wurden, als auch solche, bei denen verschiedene Kombinationen von 2 Cysteinresten bzw. alle Cysteine verändert wurden. Die exprimierten Proteine dieser Cysteinpunktmutanten weisen eine stark reduzierte bzw. fehlende Reaktivität mit IgE-Antikörpern von Allergikern auf, reagieren jedoch mit den T-Lymphozythen dieser Patienten.

Gegenstand der vorliegenden Erfindung ist daher weiterhin ein vor- oder nachstehend beschriebenes DNA-Molekül, bei dem durch ortsgerichtete Mutagenese einer, mehrere oder alle der Cystein-Reste des entsprechenden Polypeptids gegen eine andere Aminosäure ausgetauscht wurden.

Die immunmodulatorische Aktivität von hypoallergenen Fragmenten, die Polypeptiden mit T-Zell-Epitopen entsprechen, sowie die der hypoallergenen Punktmutanten (z.B. Cystein-Austausche) kann durch ihre Reaktion mit T-Zellen von Graspollenallergikem nachgewiesen werden.

Solche hypoallergenen Fragmente bzw. Punktmutanten der Cysteine können als Präparate zur Hyposensibilisierung von Allergikern eingesetzt werden, da sie mit gleicher Effektivität mit den T-Zellen reagieren, jedoch aufgrund der verminderten oder ganz fehlenden IgE-Reaktivität zu geringeren IgE-vermittelten Nebenwirkungen führen.

Werden die für die erfindungsgemäßen hypoallergenen Allergen-Varianten kodierenden Nukleinsäuren oder die unveränderten erfindungsgemäßen DNA-Moleküle mit einem humanen Expressionsvektor ligiert, können diese Konstrukte ebenfalls als Präparate für eine Immuntherapie (DNA-Vakzinierung) angewendet werden.

Schließlich sind Gegenstand der vorliegenden Erfindung pharmazeutische Zubereitungen, enthaltend mindestens ein zuvor beschriebenes DNA-Molekül oder mindestens einen zuvor beschriebenen Expressionsvektor und gegebenenfalls weitere Wirk- und/oder Hilfsstoffe zur immuntherapeutischen DNA-Vakzinierung von Patienten mit Allergien, an deren Auslösung Gruppe-4-Allergene der *Poaceae*, vorzugsweise *Triticeae*, insbesondere Sec c 4, Hor v 4, Tri a 4, beteiligt sind und/oder zur Prävention solcher Allergien.

Eine weitere Gruppe von erfindungsgemäßen pharmazeutischen Zubereitungen enthält anstelle der DNA mindestens ein zuvor beschriebenes Polypeptid und eignet sich zur Diagnose und/oder Behandlung besagter Allergien.

Pharmazeutische Zubereitungen im Sinne der vorliegenden Erfindung enthaltend als Wirkstoffe ein erfindungsgemäßes Polypeptid oder einen Expressionsvektor und/oder deren jeweilige pharmazeutisch verwendbaren Derivate, einschließlich deren Mischungen in allen Verhältnissen. Hierbei können die erfindungsgemäßen Wirkstoffe zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.
Als Hilfsstoffe sind immunstimulierende DNA oder Oligonukleotide mit CpG-Motiven besonders geeignet.

Diese Zubereitungen können als Therapeutika oder Diagnostika in der Human- oder Veterinärmedizin verwendet werden. Als Trägerstoffe kommen organische oder anorganische Substanzen in Frage, die sich für die parenterale Applikation eignen und die Wirkung des erfindungsgemäßen Wirkstoffs nicht negativ beeinflussen. Zur parenteralen Anwendung dienen insbesondere Lösungen, vorzugsweise ölige oder wässrige Lösungen, ferner Suspensionen, Emulsionen oder Implantate. Der erfindungsgemäße Wirkstoff kann auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen und/oder mehrere weitere Wirkstoffe enthalten.
Weiterhin können durch entsprechende Formulierung des erfindungsgemäßen Wirkstoffs Depotpräparate - zum Beispiel durch Adsorption an Aluminiumhydroxid - erhalten werden.

Die Erfindung dient somit auch zur Verbesserung der *in vitro* Diagnostik im Rahmen einer Allergen-Komponenten auflösenden Identifizierung des patientenspezifischen Sensibilisierungsspektrums. Die Erfindung dient ebenfalls zur Herstellung von deutlich verbesserten Präparaten zur spezifischen Immuntherapie von Gräserpollenallergien.

**Tabelle 1 Verwendete Primer**

| a) Sec c 4 | | |
|---|---|---|
| **Primer nummer** | **SEQ ID NO** | **Sequenz** |
| #0083 | 30 | GGCTCCCGGGGCGAACCAGTAG |
| #0087 | 31 | ACCAACGCCTCCCACATCCAGTC |
| #0189 | 32 | |
| #0195 | 33 | GCTCTCGATCGGCTACAATGGCG |
| #0198 | 34 | CACGCACTACAAATCTCCATGCAAG |
| #0202 | 35 | CATGCTTGATCCTTATTCTACTAGTTGGGC |
| #0203 | 36 | TACGCACGATCCTTATTCTACTAGTTGGGC |

| a) Hor v 4 | | |
|---|---|---|
| **Primer nummer** | **SEQ ID NO** | **Sequenz** |
| #0194 | 37 | GCCTTGTCCTGCCACCACGCCGCCGCCACC |
| #0195 | 38 | GCTCTCGATCGGCTACAATGGCG |
| #0198 | 39 | CACGCACTACAAATCTCCATGCAAG |
| #0202 | 40 | CATGCTTGATCCTTATTCTACTAGTTGGGC |

| c) Tri a 4 | | |
|---|---|---|
| **Primer nummer** | **SEQ ID NO** | **Sequenz** |
| #0199 | 41 | CACGCACTAAATCTCCATGCAAG |
| #0203 | 42 | TACGCACGATCCTTATTCTACTAGTTGGGC |
| #0204 | 43 | AAGCTCTATCGCCTACAATGGCG |
| #0206 | 44 | GGTGCTCCTCTTCTGCGCCTTGTCC |

## Patentansprüche

1. Ein DNA-Molekül umfassend die Nukleotidsequenz eines Getreidepollenhauptallergens gemäß SEQ ID NO 5.

2. Ein rekombinanter DNA-Expressionsvektor oder ein Klonierungssystem, enthaltend ein DNA-Molekül gemäß Anspruch 1, funktionell verbunden mit einer Expressionskontrollsequenz.

3. Ein Verfahren zur Herstellung eines Polypeptids, kodiert durch eine DNA-Sequenz gemäß Anspruch 1, durch Kultivieren eines Wirtsorganismus und Gewinnung des entsprechenden Polypeptids aus der Kultur.

4. Ein Polypeptid umfassend die Aminosäuresequenz gemäß SEQ ID NO 6, welches von einer DNA-Sequenz gemäß Anspruch 1 kodiert wird.

5. Ein Polypeptid umfassend das reife Allergen der Aminosäuresequenz gemäß SEQ ID NO 6.

6. Ein Polypeptid gemäß Anspruch 4 oder 5 als Arzneimittel.

7. Eine pharmazeutische Zubereitung, enthaltend mindestens ein Polypeptid gemäß Anspruch 6 zur Diagnose und/oder Behandlung von Allergien, an deren Auslösung Gruppe-4-Allergene der Poaceae beteiligt sind.

8. Verwendung mindestens eines Polypeptids gemäß Anspruch 6 zur Herstellung eines Arzneimittels zur Diagnose und/oder Behandlung von Allergien, an deren Auslösung Gruppe-4-Allergene der Poaceae beteiligt sind und/oder zur Prävention solcher Allergien.

9. Ein DNA-Molekül gemäß Anspruch 1 als Arzneimittel.

10. Ein rekombinanter Expressionsvektor gemäß Anspruch 2 als Arzneimittel.

11. Eine pharmazeutische Zubereitung, enthaltend mindestens ein DNA-Molekül gemäß Anspruch 9 oder mindestens einen Expressionsvektor gemäß Anspruch 10 zur immuntherapeutischen DNA-Vakzinierung von Patienten mit Allergien, an deren Auslösung Gruppe-4-Allergene der Poaceae beteiligt sind und/oder zur Prävention solcher Allergien.

12. Verwendung mindestens eines DNA-Moleküls gemäß Anspruch 9 oder mindestens eines Expressionsvektors gemäß Anspruch 10 zur Herstellung eines Arzneimittels zur immuntherapeutischen DNA-Vakzinierung von Patienten mit Allergien, an deren Auslösung Gruppe-4-Allergene der Poaceae beteiligt sind und/oder zur Prävention solcher Allergien.

## Claims

1. A DNA molecule comprising the nucleotide sequence of a cereal pollen major allergen in accordance with SEQ ID NO 5.

2. A recombinant DNA expression vector or a cloning system comprising a DNA molecule according to Claim 1, functionally connected to an expression control sequence.

3. A process for the preparation of a polypeptide encoded by a DNA sequence according to Claim 1 by cultivation of a host organism and isolation of the corresponding polypeptide from the culture.

4. A polypeptide comprising the amino acid sequence in accordance with SEQ ID NO 6, which is encoded by a DNA sequence according to Claim 1.

5. A polypeptide comprising the mature allergen of the amino acid sequence in accordance with SEQ ID NO 6.

6. A polypeptide according to Claim 4 or 5 as medicament.

7. A pharmaceutical composition comprising at least one polypeptide according to Claim 6 for the diagnosis and/or treatment of allergies in the triggering of which group 4 allergens from the Poaceae are involved.

8. Use of at least one polypeptide according to Claim 6 for the preparation of a medicament for the diagnosis and/or treatment of allergies in the triggering of which group 4 allergens from the Poaceae are involved and/or for the prevention of such allergies.

9. A DNA molecule according to Claim 1 as medicament.

10. A recombinant expression vector according to Claim 2 as medicament.

11. A pharmaceutical composition comprising at least one DNA molecule according to Claim 9 or at least one expression vector according to Claim 10 for the immunotherapeutic DNA vaccination of patients with allergies in the triggering of which group 4 allergens from the *Poaceae* are involved and/or for the prevention of such allergies.

12. Use of at least one DNA molecule according to Claim 9 or at least one expression vector according to Claim 10 for the preparation of a medicament for the immunotherapeutic DNA vaccination of patients with allergies in the triggering of which group 4 allergens from the *Poaceae* are involved and/or for the prevention of such allergies.

## Revendications

1. Molécule d'ADN comprenant la séquence de nucléotides d'un allergène majeur de pollen de céréale conformément à SEQ ID NO 5.

2. Vecteur d'expression d'ADN recombinant ou système de clonage comprenant une molécule d'ADN selon la revendication 1, fonctionnellement liée à une séquence de contrôle d'expression.

3. Procédé pour la préparation d'un polypeptide codé par une séquence d'ADN selon la revendication 1 par culture d'un organisme hôte et isolation du polypeptide correspondant vis-à-vis de la culture.

4. Polypeptide comprenant la séquence d'acides aminés conformément à SEQ ID NO 6, lequel est codé par une séquence d'ADN selon la revendication 1.

5. Polypeptide comprenant l'allergène mature de la séquence d'acides aminés conformément à SEQ ID NO 6.

6. Polypeptide selon la revendication 4 ou 5 en tant que médicament.

7. Composition pharmaceutique comprenant au moins un polypeptide selon la revendication 6 pour le diagnostic et/ou le traitement d'allergies au niveau du déclenchement desquelles des allergènes du groupe 4 issus de *Poaceae* sont mis en jeu.

8. Utilisation d'au moins un polypeptide selon la revendication 6 pour la préparation d'un médicament pour le diagnostic et/ou le traitement d'allergies au niveau du déclenchement desquelles des allergènes du groupe 4 issus de *Poaceae* sont mis en jeu et/ou pour la prévention de ces allergies.

9. Molécule d'ADN selon la revendication 1 en tant que médicament.

10. Vecteur d'expression recombinant selon la revendication 2 en tant que médicament.

11. Composition pharmaceutique comprenant au moins une molécule d'ADN selon la revendication 9 ou au moins un vecteur d'expression selon la revendication 10 pour la vaccination par ADN immunothérapeutique de patients présentant des allergies au niveau du déclenchement desquelles des allergènes du groupe 4 issus de *Poaceae* sont mis en jeu et/ou pour la prévention de ces allergies.

12. Utilisation d'au moins une molécule d'ADN selon la revendication 9 ou d'au moins un vecteur d'expression selon la revendication 10 pour la préparation d'un médicament pour la vaccination par ADN immunothérapeutique de patients présentant des allergies au niveau du déclenchement desquelles des allergènes du groupe 4 issus de *Poaceae* sont mis en jeu et/ou pour la prévention de ces allergies.


### Sequenz-Protokoll

<110> Merck Patent GmbH
<120> DNA-Sequenz und rekombinante Herstellung von Gruppe-4 Majoraller-
   genen aus Getreiden
<130> P 03/239
<140> DE 10359351.9
   <141> 2003-12-16
<160> 44
<170> PatentIn version 3.1
<210> 1
   <211> 1603
   <212> DNA
   <213> Secale cereale
<220>
   <221> stop_codon
   <222> (1555)..(1557)
   <223>
<220>
   <221> signal_sequence_DNA
   <222> (1)..(66)
   <223>
<220>
   <221> signal_sequence_PROT
   <222> (1)..(22)
   <223>
<220>
   <221> CDS
   <222> (1)..(1557)
   <223>
<400> 1
   aac tat agg gcc ttc gcg ctg gcg ctc ctc ttc tgc gcc ttg tcc tgc 48
   Asn Tyr Arg Ala Phe Ala Leu Ala Leu Leu Phe Cys Ala Leu Ser Cys
   1 5 10 15
   caa gcc gcc gcg gcc gcc tac gcg ccc gtg cct gcc aag gcg gac ttc 96
   Gln Ala Ala Ala Ala Ala Tyr Ala Pro Val Pro Ala Lys Ala Asp Phe
   20 25 30
   ctc gga tgc ctc atg aag gag ata ccg gcc cgc ctc ctc tac gcc aag 144
   Leu Gly Cys Leu Met Lys Glu Ile Pro Ala Arg Leu Leu Tyr Ala Lys
   35 40 45
   agc tcg cct gac tac ccc acc gtg ctg gcg cag acc atc agg aac tcg 192
   Ser Ser Pro Asp Tyr Pro Thr Val Leu Ala Gln Thr Ile Arg Asn Ser
   50 55 60
   cgg tgg tcg tcg ccg cag aac gtg aag ccg atc tac atc atc acc ccc 240
   Arg Trp Ser Ser Pro Gln Asn Val Lys Pro Ile Tyr Ile Ile Thr Pro
   65 70 75 80
   acc aac gcc tcg cac atc cag tcc gcg gtg gtg tgc ggc cgc cgg cac 288
   Thr Asn Ala Ser His Ile Gln Ser Ala Val Val Cys Gly Arg Arg His
   85 90 95
   ggc atc cgc ctc cgc gtg cgg agc ggc ggc cac gac tac gag ggc ctg 336
   Gly Ile Arg Leu Arg Val Arg Ser Gly Gly His Asp Tyr Glu Gly Leu
   100 105 110
   tcg tac cgg tct gag aaa ccc gag acg ttc gcc gtc gtc gac ctc aac 384
   Ser Tyr Arg Ser Glu Lys Pro Glu Thr Phe Ala Val Val Asp Leu Asn
   115 120 125
   aag atg cgg gca gtg tcg gtc gac ggc tac gcc cgc acg gcg tgg gtc 432
   Lys Met Arg Ala Val Ser Val Asp Gly Tyr Ala Arg Thr Ala Trp Val
   130 135 140
   gaa tcc ggc gcg cag ctc ggc gag ctc tac tac gcg atc gcc aag aac 480
   Glu Ser Gly Ala Gln Leu Gly Glu Leu Tyr Tyr Ala Ile Ala Lys Asn
   145 150 155 160
   agc ccc gtg ctc gcg ttc ccg gct ggc gtc tgc ccg tcc atc ggc gtc      528
   Ser Pro Val Leu Ala Phe Pro Ala Gly Val Cys Pro Ser Ile Gly Val
                   165                 170                 175
   ggc ggc aac ttc gca ggc ggc ggc ttt ggc atg ctg ctg cgc aag tac      576
   Gly Gly Asn Phe Ala Gly Gly Gly Phe Gly Met Leu Leu Arg Lys Tyr
               180                 185                 190
   ggc atc gcc gct gag aac gtc atc gac gtc aag gtg gtc gac ccc aac      624
   Gly Ile Ala Ala Glu Asn Val Ile Asp Val Lys Val Val Asp Pro Asn
           195                 200                 205
   ggc aag ctg ctc gac aag agc tcc atg agc gcg gac cac ttc tgg gcc      672
   Gly Lys Leu Leu Asp Lys Ser Ser Met Ser Ala Asp His Phe Trp Ala
       210                 215                 220
   gtt agg ggc ggc ggc gga gag agc ttt ggc atc gtc gtc tcg tgg cag      720
   Val Arg Gly Gly Gly Gly Glu Ser Phe Gly Ile Val Val Ser Trp Gln
   225                 230                 235                 240
   gtg aag ctc ctg ccg gtg cct ccc acc gtg acc gtg ctc aag atc ccc      768
   Val Lys Leu Leu Pro Val Pro Pro Thr Val Thr Val Leu Lys Ile Pro
                   245                 250                 255
   aag acg gtg caa gaa ggc gcc ata gac ctc gtc aac aag tgg cag ctg      816
   Lys Thr Val Gln Glu Gly Ala Ile Asp Leu Val Asn Lys Trp Gln Leu
               260                 265                 270
   gtc ggg ccg gca ctt ccc ggc gac ctc atg atc cgc atc atc ctt gcc      864
   Val Gly Pro Ala Leu Pro Gly Asp Leu Met Ile Arg Ile Ile Leu Ala
           275                 280                 285
   ggg aac agc gcg acg ttc gag gcc atg tac ctg ggc acc tgc agt acc      912
   Gly Asn Ser Ala Thr Phe Glu Ala Met Tyr Leu Gly Thr Cys Ser Thr
       290                 295                 300
   ctg acg ccg ctg atg agc agc aaa ttc ccc gag ctt ggc atg aac ccc      960
   Leu Thr Pro Leu Met Ser Ser Lys Phe Pro Glu Leu Gly Met Asn Pro
   305                 310                 315                 320
   tcg cac tgc aac gag atg tcc tgg atc aag tcc atc ccc ttc atc cac     1008
   Ser His Cys Asn Glu Met Ser Trp Ile Lys Ser Ile Pro Phe Ile His
                   325                 330                 335
   ctc ggc aag cag aac ctc gac gac ctc ctc aac cgg aac aac acc ttc     1056
   Leu Gly Lys Gln Asn Leu Asp Asp Leu Leu Asn Arg Asn Asn Thr Phe
               340                 345                 350
   aaa cca ttc gcc gaa tac aag tcg gac tac gtg tac cag ccc ttc ccc     1104
   Lys Pro Phe Ala Glu Tyr Lys Ser Asp Tyr Val Tyr Gln Pro Phe Pro
           355                 360                 365
   aag ccc gtg tgg gag cag atc ttc ggc tgg ctt gtg aag ccc ggc gcg     1152
   Lys Pro Val Trp Glu Gln Ile Phe Gly Trp Leu Val Lys Pro Gly Ala
       370                 375                 380
   ggg atc atg atc atg gac ccc tat ggc gcc acc atc agc gct acc ccc     1200
   Gly Ile Met Ile Met Asp Pro Tyr Gly Ala Thr Ile Ser Ala Thr Pro
   385                 390                 395                 400
   gaa gcg gcg acg ccg ttc cct cac cgc cag ggc gtc ctc ttc aac atc     1248
   Glu Ala Ala Thr Pro Phe Pro His Arg Gln Gly Val Leu Phe Asn Ile
                   405                 410                 415
   cag tac gtc aac tac tgg ttc gct gag tca gcc ggc gcg gcg ccg ctg      1296
   Gln Tyr Val Asn Tyr Trp Phe Ala Glu Ser Ala Gly Ala Ala Pro Leu
               420                 425                 430
   cag tgg agc aag gac ata tac aag ttc atg gag ccg tac gtg agc aaa      1344
   Gln Trp Ser Lys Asp Ile Tyr Lys Phe Met Glu Pro Tyr Val Ser Lys
           435                 440                 445
   aat ccc agg cag gcg tat gcc aac tac agg gac atc gac ctt ggc agg      1392
   Asn Pro Arg Gln Ala Tyr Ala Asn Tyr Arg Asp Ile Asp Leu Gly Arg
       450                 455                 460
   aat gag gtg gtg aac gac atc tcc acc tac agc agc ggc aaa gtg tgg      1440
   Asn Glu Val Val Asn Asp Ile Ser Thr Tyr Ser Ser Gly Lys Val Trp
   465                 470                 475                 480
   ggt gag aag tac ttc aag ggc aac ttc caa agg ctc gcc att acc aag      1488
   Gly Glu Lys Tyr Phe Lys Gly Asn Phe Gln Arg Leu Ala Ile Thr Lys
                   485                 490                 495
   ggc aag gtg gat cct cag gac tac ttc agg aac gag cag agc atc ccg      1536
   Gly Lys Val Asp Pro Gln Asp Tyr Phe Arg Asn Glu Gln Ser Ile Pro
               500                 505                 510
   cca ctg gtc gag aag tac tga tcgaggacct tgcatggaaa tttagtgcgt         1587
   Pro Leu Val Glu Lys Tyr
           515
   ggttggcgtt tcacat                                                    1603
<210> 2
   <211> 518
   <212> PRT
   <213> Secale cereale
<400> 2
   Asn Tyr Arg Ala Phe Ala Leu Ala Leu Leu Phe Cys Ala Leu Ser Cys
   1               5                   10                  15
   Gln Ala Ala Ala Ala Ala Tyr Ala Pro Val Pro Ala Lys Ala Asp Phe
               20                  25                  30
   Leu Gly Cys Leu Met Lys Glu Ile Pro Ala Arg Leu Leu Tyr Ala Lys
           35                  40                  45
   Ser Ser Pro Asp Tyr Pro Thr Val Leu Ala Gln Thr Ile Arg Asn Ser
       50                  55                  60
   Arg Trp Ser Ser Pro Gln Asn Val Lys Pro Ile Tyr Ile Ile Thr Pro
   65                  70                  75                  80
   Thr Asn Ala Ser His Ile Gln Ser Ala Val Val Cys Gly Arg Arg His
                   85                  90                  95
   Gly Ile Arg Leu Arg Val Arg Ser Gly Gly His Asp Tyr Glu Gly Leu
               100                 105                 110
   Ser Tyr Arg Ser Glu Lys Pro Glu Thr Phe Ala Val Val Asp Leu Asn
           115                 120                 125
   Lys Met Arg Ala Val Ser Val Asp Gly Tyr Ala Arg Thr Ala Trp Val
       130                 135                 140
   Glu Ser Gly Ala Gln Leu Gly Glu Leu Tyr Tyr Ala Ile Ala Lys Asn
   145                 150                 155                 160
   Ser Pro Val Leu Ala Phe Pro Ala Gly Val Cys Pro Ser Ile Gly Val
                   165                 170                 175
   Gly Gly Asn Phe Ala Gly Gly Gly Phe Gly Met Leu Leu Arg Lys Tyr
               180                 185                 190
   Gly Ile Ala Ala Glu Asn Val Ile Asp Val Lys Val Val Asp Pro Asn
           195                 200                 205
   Gly Lys Leu Leu Asp Lys Ser Ser Met Ser Ala Asp His Phe Trp Ala
       210                 215                 220
   Val Arg Gly Gly Gly Gly Glu Ser Phe Gly Ile Val Val Ser Trp Gln
   225                 230                 235                 240
   Val Lys Leu Leu Pro Val Pro Pro Thr Val Thr Val Leu Lys Ile Pro
                   245                 250                 255
   Lys Thr Val Gln Glu Gly Ala Ile Asp Leu Val Asn Lys Trp Gln Leu
               260                 265                 270
   Val Gly Pro Ala Leu Pro Gly Asp Leu Met Ile Arg Ile Ile Leu Ala
           275                 280                 285
   Gly Asn Ser Ala Thr Phe Glu Ala Met Tyr Leu Gly Thr Cys Ser Thr
       290                 295                 300
   Leu Thr Pro Leu Met Ser Ser Lys Phe Pro Glu Leu Gly Met Asn Pro
   305                 310                 315                 320
   Ser His Cys Asn Glu Met Ser Trp Ile Lys Ser Ile Pro Phe Ile His
                   325                 330                 335
   Leu Gly Lys Gln Asn Leu Asp Asp Leu Leu Asn Arg Asn Asn Thr Phe
               340                 345                 350
   Lys Pro Phe Ala Glu Tyr Lys Ser Asp Tyr Val Tyr Gln Pro Phe Pro
           355                 360                 365
   Lys Pro Val Trp Glu Gln Ile Phe Gly Trp Leu Val Lys Pro Gly Ala
       370                 375                 380
   Gly Ile Met Ile Met Asp Pro Tyr Gly Ala Thr Ile Ser Ala Thr Pro
   385                 390                 395                 400
   Glu Ala Ala Thr Pro Phe Pro His Arg Gln Gly Val Leu Phe Asn Ile
                   405                 410                 415
   Gln Tyr Val Asn Tyr Trp Phe Ala Glu Ser Ala Gly Ala Ala Pro Leu
               420                 425                 430
   Gln Trp Ser Lys Asp Ile Tyr Lys Phe Met Glu Pro Tyr Val Ser Lys
           435                 440                 445
   Asn Pro Arg Gln Ala Tyr Ala Asn Tyr Arg Asp Ile Asp Leu Gly Arg
       450                 455                 460
   Asn Glu Val Val Asn Asp Ile Ser Thr Tyr Ser Ser Gly Lys Val Trp
   465                 470                 475                 480
   Gly Glu Lys Tyr Phe Lys Gly Asn Phe Gln Arg Leu Ala Ile Thr Lys
                   485                 490                 495
   Gly Lys Val Asp Pro Gln Asp Tyr Phe Arg Asn Glu Gln Ser Ile Pro
               500                 505                 510
   Pro Leu Val Glu Lys Tyr
           515
<210> 3
   <211> 1644
   <212> DNA
   <213> Secale cereale
<220>
   <221> stop_codon
   <222> (1561)..(1563)
   <223>
<220>
   <221> signal_sequence_DNA
   <222> (1)..(66)
   <223>
<220>
   <221> signal_sequence_PROT
   <222> (1)..(22)
   <223>
<220>
   <221> CDS
   <222> (1)..(1563)
   <223>
<400> 3
   aac tcg agg gcc ttt gct ctg gtg ccc ctc ctc atc tgc gtc ttg tcc       48
   Asn Ser Arg Ala Phe Ala Leu Val Pro Leu Leu Ile Cys Val Leu Ser
   1               5                   10                  15
   tgc cac gcc gcc gtc tcc tac gcg gcg gcg ccg gtg ccg gcc aag gag       96
   Cys His Ala Ala Val Ser Tyr Ala Ala Ala Pro Val Pro Ala Lys Glu
               20                  25                  30
   gac ttc ttc gga tgc ctg gtg aag gag ata ccg gcc cgc ctc ctc tac      144
   Asp Phe Phe Gly Cys Leu Val Lys Glu Ile Pro Ala Arg Leu Leu Tyr
           35                  40                  45
   gcc aag agc tcg cct gcc ttc ccc acc gtc ctg gcg cag acc atc agg      192
   Ala Lys Ser Ser Pro Ala Phe Pro Thr Val Leu Ala Gln Thr Ile Arg
       50                  55                  60
   aac tcg cgg tgg tcg tcg ccg cag agc gtg aag ccg ctc tac atc atc      240
   Asn Ser Arg Trp Ser Ser Pro Gln Ser Val Lys Pro Leu Tyr Ile Ile
   65                  70                  75                  80
   acc ccc acc aac gcc tcc cac atc cag tcc gcg gtg gtg tgc ggc cgc      288
   Thr Pro Thr Asn Ala Ser His Ile Gln Ser Ala Val Val Cys Gly Arg
                   85                  90                  95
   cgg cac ggc gtc cgc atc cgc gtg cgg agc ggc ggc cac gac tac gag      336
   Arg His Gly Val Arg Ile Arg Val Arg Ser Gly Gly His Asp Tyr Glu
               100                 105                 110
   ggc ctg tcg tac cgg tcc gag cgc ccc gag gcg ttc gcc gtc gtc gac      384
   Gly Leu Ser Tyr Arg Ser Glu Arg Pro Glu Ala Phe Ala Val Val Asp
           115                 120                 125
   ctc aac aag atg cgg gcc gtg gtg gtc gac ggc aag gct cgc acg gcg      432
   Leu Asn Lys Met Arg Ala Val Val Val Asp Gly Lys Ala Arg Thr Ala
       130                 135                 140
   tgg gtg gac tcc ggt gcg cag ctc ggc gag ctc tac tac gcc atc gcc      480
   Trp Val Asp Ser Gly Ala Gln Leu Gly Glu Leu Tyr Tyr Ala Ile Ala
   145                 150                 155                 160
   aag aac agc ccc gtg ctc gcg ttc ccg gcc ggc gtt tgc ccg acc att      528
   Lys Asn Ser Pro Val Leu Ala Phe Pro Ala Gly Val Cys Pro Thr Ile
                   165                 170                 175
   ggt gta ggc ggc aac ttc gct ggc ggc ggc ttc ggc atg ctg ctg cgc      576
   Gly Val Gly Gly Asn Phe Ala Gly Gly Gly Phe Gly Met Leu Leu Arg
               180                 185                 190
   aag tac ggc atc gcc gcc gag aac gtc atc gac gtg aag gtg gtc gac      624
   Lys Tyr Gly Ile Ala Ala Glu Asn Val Ile Asp Val Lys Val Val Asp
           195                 200                 205
   gcc aac ggc aca ctg ctc gac aag agc tcc atg agc gcg gat cac ttc      672
   Ala Asn Gly Thr Leu Leu Asp Lys Ser Ser Met Ser Ala Asp His Phe
       210                 215                 220
   tgg gcc gtc agg ggc ggc ggc gga gag agc ttc ggc atc gtc gtg tcg      720
   Trp Ala Val Arg Gly Gly Gly Gly Glu Ser Phe Gly Ile Val Val Ser
   225                 230                 235                 240
   tgg cag gtg aag ctc ctc ccg gtg cct ccc acc gtg acc gtg ttc aag      768
   Trp Gln Val Lys Leu Leu Pro Val Pro Pro Thr Val Thr Val Phe Lys
                   245                 250                 255
   atc ccc aag acg gtg caa gaa ggc gcc gta gag ctc atc aac aag tgg      816
   Ile Pro Lys Thr Val Gln Glu Gly Ala Val Glu Leu Ile Asn Lys Trp
               260                 265                 270
   cag cta gtc gcg ccg gcc ctc ccc gac gac ctg atg atc cgc atc atc      864
   Gln Leu Val Ala Pro Ala Leu Pro Asp Asp Leu Met Ile Arg Ile Ile
           275                 280                 285
   gct ttc ggc ggc acc gcc aag ttc gag gcc atg tac ctg ggc acc tgc      912
   Ala Phe Gly Gly Thr Ala Lys Phe Glu Ala Met Tyr Leu Gly Thr Cys
       290                 295                 300
   aaa gcc ctg aca ccg ctg atg agc agc aga ttc ccc gag ctc ggc atg      960
   Lys Ala Leu Thr Pro Leu Met Ser Ser Arg Phe Pro Glu Leu Gly Met
   305                 310                 315                 320
   aac gcc tcg cac tgc aac gag atg ccc tgg atc aag tcc gtc cca ttc      1008
   Asn Ala Ser His Cys Asn Glu Met Pro Trp Ile Lys Ser Val Pro Phe
                   325                 330                 335
   atc cac ctt ggc aag cag gcc acc ctc tcc gac ctc ctc aac cgg aac    1056
   Ile His Leu Gly Lys Gln Ala Thr Leu Ser Asp Leu Leu Asn Arg Asn
               340                 345                 350
   aac acc ttc aaa ccc ttc gcc gag tac aag tcg gac tac gtc tac cag    1104
   Asn Thr Phe Lys Pro Phe Ala Glu Tyr Lys Ser Asp Tyr Val Tyr Gln
           355                 360                 365
   ccc gtc ccc aag ccc gtc tgg gcg cag atc ttc gtc tgg ctc gtc aaa    1152
   Pro Val Pro Lys Pro Val Trp Ala Gln Ile Phe Val Trp Leu Val Lys
       370                 375                 380
   ccc ggc gcc ggg atc atg gtc atg gac ccc tac ggc gcc gcc atc agc    1200
   Pro Gly Ala Gly Ile Met Val Met Asp Pro Tyr Gly Ala Ala Ile Ser
   385                 390                 395                 400
   gcc acc ccc gaa gcc gcc acg ccg ttc cct cac cgc aag gac gtc ctc    1248
   Ala Thr Pro Glu Ala Ala Thr Pro Phe Pro His Arg Lys Asp Val Leu
                   405                 410                 415
   ttc aac atc cag tac gtc aac tac tgg ttc gac gag gca ggc ggc gcc    1296
   Phe Asn Ile Gln Tyr Val Asn Tyr Trp Phe Asp Glu Ala Gly Gly Ala
               420                 425                 430
   gcg ccg ctg cag tgg agc aag gac atg tac agg ttc atg gag ccg tac    1344
   Ala Pro Leu Gln Trp Ser Lys Asp Met Tyr Arg Phe Met Glu Pro Tyr
           435                 440                 445
   gtc agc aag aac ccc aga cag gcc tac gcc aac tac agg gac atc gac    1392
   Val Ser Lys Asn Pro Arg Gln Ala Tyr Ala Asn Tyr Arg Asp Ile Asp
       450                 455                 460
   ctc ggc agg aac gag gtg gtc aac gac atc tcc acc tat gcc agc ggc    1440
   Leu Gly Arg Asn Glu Val Val Asn Asp Ile Ser Thr Tyr Ala Ser Gly
   465                 470                 475                 480
   aag gtc tgg ggc gag aag tac ttc aag ggc aac ttc caa agg ctc gcc    1488
   Lys Val Trp Gly Glu Lys Tyr Phe Lys Gly Asn Phe Gln Arg Leu Ala
                   485                 490                 495
   att acc aag ggc aag gtg gat cct cag gac tac ttc agg aac gag cag    1536
   Ile Thr Lys Gly Lys Val Asp Pro Gln Asp Tyr Phe Arg Asn Glu Gln
               500                 505                 510
   agc atc ccg ccg ctg cta ggg aag tag tagtactctt gcttgcatgg          1583
   Ser Ile Pro Pro Leu Leu Gly Lys
           515                 520
   agatttgtag tgcgtctttc gcgtttcaaa tgcccaacta gtagaataag gatcgtgcgt  1643
   a                                                                  1644
<210> 4
   <211> 520
   <212> PRT
   <213> Secale cereale
<400> 4
   Asn Ser Arg Ala Phe Ala Leu Val Pro Leu Leu Ile Cys Val Leu Ser
   1               5                   10                  15
   Cys His Ala Ala Val Ser Tyr Ala Ala Ala Pro Val Pro Ala Lys Glu
               20                  25                  30
   Asp Phe Phe Gly Cys Leu Val Lys Glu Ile Pro Ala Arg Leu Leu Tyr
           35                  40                  45
   Ala Lys Ser Ser Pro Ala Phe Pro Thr Val Leu Ala Gln Thr Ile Arg
       50                  55                  60
   Asn Ser Arg Trp Ser Ser Pro Gln Ser Val Lys Pro Leu Tyr Ile Ile
   65                  70                  75                  80
   Thr Pro Thr Asn Ala Ser His Ile Gln Ser Ala Val Val Cys Gly Arg
                   85                  90                  95
   Arg His Gly Val Arg Ile Arg Val Arg Ser Gly Gly His Asp Tyr Glu
               100                 105                 110
   Gly Leu Ser Tyr Arg Ser Glu Arg Pro Glu Ala Phe Ala Val Val Asp
           115                 120                 125
   Leu Asn Lys Met Arg Ala Val Val Val Asp Gly Lys Ala Arg Thr Ala
       130                 135                 140
   Trp Val Asp Ser Gly Ala Gln Leu Gly Glu Leu Tyr Tyr Ala Ile Ala
   145                 150                 155                 160
   Lys Asn Ser Pro Val Leu Ala Phe Pro Ala Gly Val Cys Pro Thr Ile
                   165                 170                 175
   Gly Val Gly Gly Asn Phe Ala Gly Gly Gly Phe Gly Met Leu Leu Arg
               180                 185                 190
   Lys Tyr Gly Ile Ala Ala Glu Asn Val Ile Asp Val Lys Val Val Asp
           195                 200                 205
   Ala Asn Gly Thr Leu Leu Asp Lys Ser Ser Met Ser Ala Asp His Phe
       210                 215                 220
   Trp Ala Val Arg Gly Gly Gly Gly Glu Ser Phe Gly Ile Val Val Ser
   225                 230                 235                 240
   Trp Gln Val Lys Leu Leu Pro Val Pro Pro Thr Val Thr Val Phe Lys
                   245                 250                 255
   Ile Pro Lys Thr Val Gln Glu Gly Ala Val Glu Leu Ile Asn Lys Trp
               260                 265                 270
   Gln Leu Val Ala Pro Ala Leu Pro Asp Asp Leu Met Ile Arg Ile Ile
           275                 280                 285
   Ala Phe Gly Gly Thr Ala Lys Phe Glu Ala Met Tyr Leu Gly Thr Cys
       290                 295                 300
   Lys Ala Leu Thr Pro Leu Met Ser Ser Arg Phe Pro Glu Leu Gly Met
   305                 310                 315                 320
   Asn Ala Ser His Cys Asn Glu Met Pro Trp Ile Lys Ser Val Pro Phe
                   325                 330                 335
   Ile His Leu Gly Lys Gln Ala Thr Leu Ser Asp Leu Leu Asn Arg Asn
               340                 345                 350
   Asn Thr Phe Lys Pro Phe Ala Glu Tyr Lys Ser Asp Tyr Val Tyr Gln
           355                 360                 365
   Pro Val Pro Lys Pro Val Trp Ala Gln Ile Phe Val Trp Leu Val Lys
       370                 375                 380
   Pro Gly Ala Gly Ile Met Val Met Asp Pro Tyr Gly Ala Ala Ile Ser
   385                 390                 395                 400
   Ala Thr Pro Glu Ala Ala Thr Pro Phe Pro His Arg Lys Asp Val Leu
                   405                 410                 415
   Phe Asn Ile Gln Tyr Val Asn Tyr Trp Phe Asp Glu Ala Gly Gly Ala
               420                 425                 430
   Ala Pro Leu Gln Trp Ser Lys Asp Met Tyr Arg Phe Met Glu Pro Tyr
           435                 440                 445
   Val Ser Lys Asn Pro Arg Gln Ala Tyr Ala Asn Tyr Arg Asp Ile Asp
       450                 455                 460
   Leu Gly Arg Asn Glu Val Val Asn Asp Ile Ser Thr Tyr Ala Ser Gly
   465                 470                 475                 480
   Lys Val Trp Gly Glu Lys Tyr Phe Lys Gly Asn Phe Gln Arg Leu Ala
                   485                 490                     495
   Ile Thr Lys Gly Lys Val Asp Pro Gln Asp Tyr Phe Arg Asn Glu Gln
               500                 505                 510
   Ser Ile Pro Pro Leu Leu Gly Lys
           515                 520
<210> 5
   <211> 1608
   <212> DNA
   <213> Hordeum vulgare
<220>
   <221> stop_codon
   <222> (1555)..(1557)
   <223>
<220>
   <221> signal_sequence_DNA
   <222> (1)..(66)
   <223>
<220>
   <221> signal_sequence_PROT
   <222> (1)..(22)
   <223>
<220>
   <221> CDS
   <222> (1)..(1557)
   <223>
<400> 5
   agc tcg agg gcc ttc gct ctg gtg ctc ctc ctc tgc gcc ttg tcc tgc      48
   Ser Ser Arg Ala Phe Ala Leu Val Leu Leu Leu Cys Ala Leu Ser Cys
   1               5                   10                  15
   cac cac gct gcc gtc tcc tcc gcg cag gtg ccg gcc aag gac gac ttc      96
   His His Ala Ala Val Ser Ser Ala Gln Val Pro Ala Lys Asp Asp Phe
               20                  25                  30
   ctg gga tgc ctc gtg aag gag ata ccg gcc cgc ctc ctc ttc gcc aag     144
   Leu Gly Cys Leu Val Lys Glu Ile Pro Ala Arg Leu Leu Phe Ala Lys
           35                  40                  45
   agc tcg cct gcc ttc ccc gcc gtc ctg gag cag acc atc agg aac tcg     192
   Ser Ser Pro Ala Phe Pro Ala Val Leu Glu Gln Thr Ile Arg Asn Ser
       50                  55                  60
   cgg tgg tcg tcg ccg cag aac gtg aag ccg ctc tac atc atc acc ccc     240
   Arg Trp Ser Ser Pro Gln Asn Val Lys Pro Leu Tyr Ile Ile Thr Pro
   65                  70                  75                  80
   acc aac acc tcc cac atc cag tct gct gtg gtg tgc ggc cgc cgg cac     288
   Thr Asn Thr Ser His Ile Gln Ser Ala Val Val Cys Gly Arg Arg His
                   85                  90                  95
   ggc gtc cgc ctc cgc gtg cgg agc ggc ggc cac gac tac gag ggc ctg     336
   Gly Val Arg Leu Arg Val Arg Ser Gly Gly His Asp Tyr Glu Gly Leu
               100                 105                 110
   tcg tac cgg tcc gag cgc ccc gag gcg ttc gcc gtc gta gac ctc aac     384
   Ser Tyr Arg Ser Glu Arg Pro Glu Ala Phe Ala Val Val Asp Leu Asn
           115                 120                 125
   aag atg cgg acc gtg ttg gtc aac gaa aag gcc cgc acg gcg tgg gtg     432
   Lys Met Arg Thr Val Leu Val Asn Glu Lys Ala Arg Thr Ala Trp Val
       130                 135                 140
   gac tcc ggc gcg cag ctc ggc gag ctc tac tac gcc atc gcc aag aac     480
   Asp Ser Gly Ala Gln Leu Gly Glu Leu Tyr Tyr Ala Ile Ala Lys Asn
   145                 150                 155                 160
   agc ccc gtg ctc gcg ttc cca gcc ggc gtt tgc ccg tcc att ggt gta     528
   Ser Pro Val Leu Ala Phe Pro Ala Gly Val Cys Pro Ser Ile Gly Val
                   165                 170                 175
   ggt ggc aac ttc gct ggc ggc ggc ttc ggc atg ctg ctg cgc aag tac     576
   Gly Gly Asn Phe Ala Gly Gly Gly Phe Gly Met Leu Leu Arg Lys Tyr
               180                 185                 190
   ggc atc gcc gcc gag aac gtc atc gac gtc aag ctg gtc gac gcc aac     624
   Gly Ile Ala Ala Glu Asn Val Ile Asp Val Lys Leu Val Asp Ala Asn
           195                 200                 205
   ggc aag ctg ctc gac aag agc tcc atg agc ccg gac cac ttc tgg gcc     672
   Gly Lys Leu Leu Asp Lys Ser Ser Met Ser Pro Asp His Phe Trp Ala
       210                 215                 220
   gtc agg ggc ggc ggc gga gag agc ttc ggc atc gtc gtc tcg tgg cag     720
   Val Arg Gly Gly Gly Gly Glu Ser Phe Gly Ile Val Val Ser Trp Gln
   225                 230                 235                 240
   gtg aag ctt ctc ccg gtg cct ccc acc gtg act gtg ttt cag atc ccc      768
   Val Lys Leu Leu Pro Val Pro Pro Thr Val Thr Val Phe Gln Ile Pro
                   245                 250                 255
   aag aca gtg caa gaa ggc gcc gta gac ctc atc aac aag tgg cag ctg      816
   Lys Thr Val Gln Glu Gly Ala Val Asp Leu Ile Asn Lys Trp Gln Leu
               260                 265                 270
   gtc gcg ccg gcc ctt ccc ggc gac atc atg atc cgc atc atc gcc atg      864
   Val Ala Pro Ala Leu Pro Gly Asp Ile Met Ile Arg Ile Ile Ala Met
           275                 280                 285
   ggg gac aaa gcg acg ttc gag gcc atg tac ctg ggc acc tgc aaa acc      912
   Gly Asp Lys Ala Thr Phe Glu Ala Met Tyr Leu Gly Thr Cys Lys Thr
       290                 295                 300
   ctg acg ccg ctg atg agc agc aaa ttc ccg gag ctt ggc atg aac ccc      960
   Leu Thr Pro Leu Met Ser Ser Lys Phe Pro Glu Leu Gly Met Asn Pro
   305                 310                 315                 320
   tcg cac tgc aac gag atg ccc tgg atc aag tcc atc ccc ttc atc cac     1008
   Ser His Cys Asn Glu Met Pro Trp Ile Lys Ser Ile Pro Phe Ile His
                   325                 330                 335
   ctt ggc aag cag gcc acc ctg gcc gac ctc ctc aac cgg aac aac acc     1056
   Leu Gly Lys Gln Ala Thr Leu Ala Asp Leu Leu Asn Arg Asn Asn Thr
               340                 345                 350
   ttc aaa ccc ttc gcc gaa tac aag tcg gac tac gtc tac cag ccc gtc     1104
   Phe Lys Pro Phe Ala Glu Tyr Lys Ser Asp Tyr Val Tyr Gln Pro Val
           355                 360                 365
   ccc aag ccc gtg tgg gag cag ctc ttc ggc tgg ctc acg aaa ccc ggc     1152
   Pro Lys Pro Val Trp Glu Gln Leu Phe Gly Trp Leu Thr Lys Pro Gly
       370                 375                 380
   gcg ggg atc atg gtc atg gac cca tac ggc gcc acc atc agc gcc acc     1200
   Ala Gly Ile Met Val Met Asp Pro Tyr Gly Ala Thr Ile Ser Ala Thr
   385                 390                 395                 400
   ccc gaa gcg gcg acg ccg ttc cct cac cgc aag ggc gtc ctc ttc aac     1248
   Pro Glu Ala Ala Thr Pro Phe Pro His Arg Lys Gly Val Leu Phe Asn
                   405                 410                 415
   atc cag tac gtc aac tac tgg ttc gcc gag gca gcc ggc gcc gcg ccg     1296
   Ile Gln Tyr Val Asn Tyr Trp Phe Ala Glu Ala Ala Gly Ala Ala Pro
               420                 425                 430
   ctg cag tgg agc aag gac att tac aaa ttc atg gag ccg ttc gtg agc     1344
   Leu Gln Trp Ser Lys Asp Ile Tyr Lys Phe Met Glu Pro Phe Val Ser
           435                 440                 445
   aag aac ccc agg cag gcg tac gcc aac tac agg gac atc gac ctc ggc     1392
   Lys Asn Pro Arg Gln Ala Tyr Ala Asn Tyr Arg Asp Ile Asp Leu Gly
       450                 455                 460
   agg aac gag gtg gtg aac gac atc tca acc tac agc agc ggc aag gtg     1440
   Arg Asn Glu Val Val Asn Asp Ile Ser Thr Tyr Ser Ser Gly Lys Val
   465                 470                 475                 480
   tgg ggc gag aag tac ttc aag ggc aac ttc caa agg ctc gcc atc acc     1488
   Trp Gly Glu Lys Tyr Phe Lys Gly Asn Phe Gln Arg Leu Ala Ile Thr
                   485                 490                 495
   aag ggc aag gtg gat ccc cag gac tac ttc agg aac gag cag agc atc      1536
   Lys Gly Lys Val Asp Pro Gln Asp Tyr Phe Arg Asn Glu Gln Ser Ile
               500                 505                 510
   ccg ccg ctg ctg ggc aag tag tgaccgagag tcttgcatgg agatttgtag        1587
   Pro Pro Leu Leu Gly Lys
           515
   tgcgtgcttg gcgtttctga t                                              1608
<210> 6
   <211> 518
   <212> PRT
   <213> Hordeum vulgare
<400> 6
   Ser Ser Arg Ala Phe Ala Leu Val Leu Leu Leu Cys Ala Leu Ser Cys
   1               5                   10                  15
   His His Ala Ala Val Ser Ser Ala Gln Val Pro Ala Lys Asp Asp Phe
               20                  25                  30
   Leu Gly Cys Leu Val Lys Glu Ile Pro Ala Arg Leu Leu Phe Ala Lys
           35                  40                  45
   Ser Ser Pro Ala Phe Pro Ala Val Leu Glu Gln Thr Ile Arg Asn Ser
       50                  55                  60
   Arg Trp Ser Ser Pro Gln Asn Val Lys Pro Leu Tyr Ile Ile Thr Pro
   65                  70                  75                  80
   Thr Asn Thr Ser His Ile Gln Ser Ala Val Val Cys Gly Arg Arg His
                   85                  90                  95
   Gly Val Arg Leu Arg Val Arg Ser Gly Gly His Asp Tyr Glu Gly Leu
               100                 105                 110
   Ser Tyr Arg Ser Glu Arg Pro Glu Ala Phe Ala Val Val Asp Leu Asn
           115                 120                 125
   Lys Met Arg Thr Val Leu Val Asn Glu Lys Ala Arg Thr Ala Trp Val
       130                 135                 140
   Asp Ser Gly Ala Gln Leu Gly Glu Leu Tyr Tyr Ala Ile Ala Lys Asn
   145                 150                 155                 160
   Ser Pro Val Leu Ala Phe Pro Ala Gly Val Cys Pro Ser Ile Gly Val
                   165                 170                 175
   Gly Gly Asn Phe Ala Gly Gly Gly Phe Gly Met Leu Leu Arg Lys Tyr
               180                 185                 190
   Gly Ile Ala Ala Glu Asn Val Ile Asp Val Lys Leu Val Asp Ala Asn
           195                 200                 205
   Gly Lys Leu Leu Asp Lys Ser Ser Met Ser Pro Asp His Phe Trp Ala
       210                 215                 220
   Val Arg Gly Gly Gly Gly Glu Ser Phe Gly Ile Val Val Ser Trp Gln
   225                 230                 235                 240
   Val Lys Leu Leu Pro Val Pro Pro Thr Val Thr Val Phe Gln Ile Pro
                   245                 250                 255
   Lys Thr Val Gln Glu Gly Ala Val Asp Leu Ile Asn Lys Trp Gln Leu
               260                 265                 270
   Val Ala Pro Ala Leu Pro Gly Asp Ile Met Ile Arg Ile Ile Ala Met
           275                 280                 285
   Gly Asp Lys Ala Thr Phe Glu Ala Met Tyr Leu Gly Thr Cys Lys Thr
       290                 295                 300
   Leu Thr Pro Leu Met Ser Ser Lys Phe Pro Glu Leu Gly Met Asn Pro
   305                 310                 315                 320
   Ser His Cys Asn Glu Met Pro Trp Ile Lys Ser Ile Pro Phe Ile His
                   325                 330                 335
   Leu Gly Lys Gln Ala Thr Leu Ala Asp Leu Leu Asn Arg Asn Asn Thr
               340                 345                 350
   Phe Lys Pro Phe Ala Glu Tyr Lys Ser Asp Tyr Val Tyr Gln Pro Val
           355                 360                 365
   Pro Lys Pro Val Trp Glu Gln Leu Phe Gly Trp Leu Thr Lys Pro Gly
       370                 375                 380
   Ala Gly Ile Met Val Met Asp Pro Tyr Gly Ala Thr Ile Ser Ala Thr
   385                 390                 395                 400
   Pro Glu Ala Ala Thr Pro Phe Pro His Arg Lys Gly Val Leu Phe Asn
   405 410 415
   Ile Gln Tyr Val Asn Tyr Trp Phe Ala Glu Ala Ala Gly Ala Ala Pro
   420 425 430
   Leu Gln Trp Ser Lys Asp Ile Tyr Lys Phe Met Glu Pro Phe Val Ser
   435 440 445
   Lys Asn Pro Arg Gln Ala Tyr Ala Asn Tyr Arg Asp Ile Asp Leu Gly
   450 455 460
   Arg Asn Glu Val Val Asn Asp Ile Ser Thr Tyr Ser Ser Gly Lys Val
   465 470 475 480
   Trp Gly Glu Lys Tyr Phe Lys Gly Asn Phe Gln Arg Leu Ala Ile Thr
   485 490 495
   Lys Gly Lys Val Asp Pro Gln Asp Tyr Phe Arg Asn Glu Gln Ser Ile
   500 505 510
   Pro Pro Leu Leu Gly Lys
   515
<210> 7
   <211> 1603
   <212> DNA
   <213> Triticum aestivum
<220>
   <221> stop_codon
   <222> (1555)..(1557)
   <223>
<220>
   <221> signal_sequence_DNA
   <222> (1)..(63)
   <223>
<220>
   <221> signal_sequence_PROT
   <222> (1)..(21)
   <223>
<220>
   <221> CDS
   <222> (1)..(1557)
   <223>
<400> 7
   aac tat agg gcc ttc acg ctg gtg ctc ctc ttc tgc gcc ttg tcc tgt      48
   Asn Tyr Arg Ala Phe Thr Leu Val Leu Leu Phe Cys Ala Leu Ser Cys
   1               5                   10                  15
   caa gcc gcc gcc acc tac gcg ccg gtg cct gcc aag gag gac ttc ctc      96
   Gln Ala Ala Ala Thr Tyr Ala Pro Val Pro Ala Lys Glu Asp Phe Leu
               20                  25                  30
   gga tgc ctc atg aag gag ata ccg gca cgc ctc ctc tac gcc aag agc      144
   Gly Cys Leu Met Lys Glu Ile Pro Ala Arg Leu Leu Tyr Ala Lys Ser
           35                  40                  45
   tcg cct gac ttc ccc acc gtc ctg gcg cag acc atc agg aac tcg cgg      192
   Ser Pro Asp Phe Pro Thr Val Leu Ala Gln Thr Ile Arg Asn Ser Arg
       50                  55                  60
   tgg ttg tcg ccg cag aac gtg aag ccg ctc tac atc atc acc ccc acc      240
   Trp Leu Ser Pro Gln Asn Val Lys Pro Leu Tyr Ile Ile Thr Pro Thr
   65                  70                  75                  80
   aac gcc tcg cac atc cag tcc gcg gtg gtg tgc gga cgc cgg cac agc      288
   Asn Ala Ser His Ile Gln Ser Ala Val Val Cys Gly Arg Arg His Ser
                   85                  90                  95
   gtc cgc ctc cgc gtc cgg agc ggc ggc cac gac tac gag ggc ctg tcg      336
   Val Arg Leu Arg Val Arg Ser Gly Gly His Asp Tyr Glu Gly Leu Ser
               100                 105                 110
   tac cgg tcc gag aaa ccc gag acg ttc gcc gtc gtc gac ctc aac aag      384
   Tyr Arg Ser Glu Lys Pro Glu Thr Phe Ala Val Val Asp Leu Asn Lys
           115                 120                 125
   atg cgg gca gtg ttg atc gac ggc tac gcc cgc acg gcg tgg gtc gaa      432
   Met Arg Ala Val Leu Ile Asp Gly Tyr Ala Arg Thr Ala Trp Val Glu
       130                 135                 140
   tcc ggc gcg cag ctc ggc gag ctc tac tac gcc atc gcg aaa aac agc      480
   Ser Gly Ala Gln Leu Gly Glu Leu Tyr Tyr Ala Ile Ala Lys Asn Ser
   145                 150                 155                 160
   ccc gtg ctc gcg ttc ccg gcc ggc gtc tgc ccg acc atc ggc gtc ggc      528
   Pro Val Leu Ala Phe Pro Ala Gly Val Cys Pro Thr Ile Gly Val Gly
                   165                 170                 175
   ggc aac ttc gca ggc ggc ggc ttt ggc atg ctg ctg cgg aag tac ggc      576
   Gly Asn Phe Ala Gly Gly Gly Phe Gly Met Leu Leu Arg Lys Tyr Gly
               180                 185                 190
   atc gcc gcc gag aac gtc atc gac gtc aag gtg gtc gac ccc aac ggc      624
   Ile Ala Ala Glu Asn Val Ile Asp Val Lys Val Val Asp Pro Asn Gly
           195                 200                 205
   aag ctt ctc gac aag agc tcc atg agc ccg gac cac ttc tgg gcc gtc      672
   Lys Leu Leu Asp Lys Ser Ser Met Ser Pro Asp His Phe Trp Ala Val
       210                 215                 220
   agg ggc ggc ggc gga gag agc ttt ggc atc gtc gtg tcg tgg caa gtg      720
   Arg Gly Gly Gly Gly Glu Ser Phe Gly Ile Val Val Ser Trp Gln Val
   225                 230                 235                 240
   aag ctc ctg ccg gtg cct ccc acc gtg acc gtg ttc aag atc ccc aag      768
   Lys Leu Leu Pro Val Pro Pro Thr Val Thr Val Phe Lys Ile Pro Lys
                   245                 250                 255
   aca gtg caa gaa ggc gcc gta gac ctc gtc aac aag tgg caa ctg gtc      816
   Thr Val Gln Glu Gly Ala Val Asp Leu Val Asn Lys Trp Gln Leu Val
               260                 265                 270
   ggg ccg gcc ctt ccc ggc gac ctc atg atc cgc gtc atc gct gcg ggg      864
   Gly Pro Ala Leu Pro Gly Asp Leu Met Ile Arg Val Ile Ala Ala Gly
           275                 280                 285
   aac acc gcg aca ttc gag ggc atg tac ctg ggc acc tgc caa acc ctg      912
   Asn Thr Ala Thr Phe Glu Gly Met Tyr Leu Gly Thr Cys Gln Thr Leu
       290                 295                 300
   acg ccg ttg atg agc agc caa ttc ccc gag ctt ggc atg aac ccc tat      960
   Thr Pro Leu Met Ser Ser Gln Phe Pro Glu Leu Gly Met Asn Pro Tyr
   305                 310                 315                 320
   cac tgc aac gag atg ccc tgg atc aag tcc atc ccc ttc atc cac ctc     1008
   His Cys Asn Glu Met Pro Trp Ile Lys Ser Ile Pro Phe Ile His Leu
                   325                 330                 335
   ggc aaa gag gcc agc ctg gtc gac ctc ctc aac cgg aac aac acc ttc     1056
   Gly Lys Glu Ala Ser Leu Val Asp Leu Leu Asn Arg Asn Asn Thr Phe
               340                 345                 350
   aag ccc ttc gcc gaa tac aag tcg gac tac gtg tac cag ccc ttc ccc     1104
   Lys Pro Phe Ala Glu Tyr Lys Ser Asp Tyr Val Tyr Gln Pro Phe Pro
           355                 360                 365
   aag ccc gtg tgg gag cag atc ttc ggc tgg ctc acg aag ccc ggt ggg     1152
   Lys Pro Val Trp Glu Gln Ile Phe Gly Trp Leu Thr Lys Pro Gly Gly
       370                 375                 380
   ggg atg atg atc atg gac cca tac ggc gcc acc atc agc gcc acc ccc     1200
   Gly Met Met Ile Met Asp Pro Tyr Gly Ala Thr Ile Ser Ala Thr Pro
   385                 390                 395                 400
   gaa gcg gcg acg ccg ttc cct cac cgc cag ggc gtt ctc ttc aac atc     1248
   Glu Ala Ala Thr Pro Phe Pro His Arg Gln Gly Val Leu Phe Asn Ile
                   405                 410                 415
   cag tac gtc aac tac tgg ttc gcc gag gca gcc gcc gcc gcg ccg ctg      1296
   Gln Tyr Val Asn Tyr Trp Phe Ala Glu Ala Ala Ala Ala Ala Pro Leu
               420                 425                 430
   cag tgg agc aag gac atg tac aat ttc atg gag ccg tac gtg agc aag      1344
   Gln Trp Ser Lys Asp Met Tyr Asn Phe Met Glu Pro Tyr Val Ser Lys
           435                 440                 445
   aac ccc agg cag gcg tac gcc aac tac agg gac att gac ctc ggc agg      1392
   Asn Pro Arg Gln Ala Tyr Ala Asn Tyr Arg Asp Ile Asp Leu Gly Arg
       450                 455                 460
   aac gag gtg gtg aac gac atc tca acc tat agc agc ggc aag gtt tgg      1440
   Asn Glu Val Val Asn Asp Ile Ser Thr Tyr Ser Ser Gly Lys Val Trp
   465                 470                 475                 480
   ggc gag aag tac ttc aag ggc aac ttc caa agg ctc gct att acc aag      1488
   Gly Glu Lys Tyr Phe Lys Gly Asn Phe Gln Arg Leu Ala Ile Thr Lys
                   485                 490                 495
   ggc aag gtg gat cct cag gac tac ttc agg aac gag cag agc atc ccg      1536
   Gly Lys Val Asp Pro Gln Asp Tyr Phe Arg Asn Glu Gln Ser Ile Pro
               500                 505                 510
   ccg ctg ctc gag aag tac tga tcgaggacct tgcatggaga tttagtgcgt        1587
   Pro Leu Leu Glu Lys Tyr
           515
   ggttgccgtt tcacat                                                   1603
<210> 8
   <211> 518
   <212> PRT
   <213> Triticum aestivum
<400> 8
   Asn Tyr Arg Ala Phe Thr Leu Val Leu Leu Phe Cys Ala Leu Ser Cys
   1               5                   10                  15
   Gln Ala Ala Ala Thr Tyr Ala Pro Val Pro Ala Lys Glu Asp Phe Leu
               20                  25                  30
   Gly Cys Leu Met Lys Glu Ile Pro Ala Arg Leu Leu Tyr Ala Lys Ser
           35                  40                  45
   Ser Pro Asp Phe Pro Thr Val Leu Ala Gln Thr Ile Arg Asn Ser Arg
       50                  55                  60
   Trp Leu Ser Pro Gln Asn Val Lys Pro Leu Tyr Ile Ile Thr Pro Thr
   65                  70                  75                  80
   Asn Ala Ser His Ile Gln Ser Ala Val Val Cys Gly Arg Arg His Ser
                   85                  90                  95
   Val Arg Leu Arg Val Arg Ser Gly Gly His Asp Tyr Glu Gly Leu Ser
               100                 105                 110
   Tyr Arg Ser Glu Lys Pro Glu Thr Phe Ala Val Val Asp Leu Asn Lys
           115                 120                 125
   Met Arg Ala Val Leu Ile Asp Gly Tyr Ala Arg Thr Ala Trp Val Glu
       130                 135                 140
   Ser Gly Ala Gln Leu Gly Glu Leu Tyr Tyr Ala Ile Ala Lys Asn Ser
   145                 150                 155                 160
   Pro Val Leu Ala Phe Pro Ala Gly Val Cys Pro Thr Ile Gly Val Gly
                   165                 170                 175
   Gly Asn Phe Ala Gly Gly Gly Phe Gly Met Leu Leu Arg Lys Tyr Gly
               180                 185                 190
   Ile Ala Ala Glu Asn Val Ile Asp Val Lys Val Val Asp Pro Asn Gly
           195                 200                 205
   Lys Leu Leu Asp Lys Ser Ser Met Ser Pro Asp His Phe Trp Ala Val
       210                 215                 220
   Arg Gly Gly Gly Gly Glu Ser Phe Gly Ile Val Val Ser Trp Gln Val
   225                 230                 235                 240
   Lys Leu Leu Pro Val Pro Pro Thr Val Thr Val Phe Lys Ile Pro Lys
                   245                 250                 255
   Thr Val Gln Glu Gly Ala Val Asp Leu Val Asn Lys Trp Gln Leu Val
               260                 265                 270
   Gly Pro Ala Leu Pro Gly Asp Leu Met Ile Arg Val Ile Ala Ala Gly
           275                 280                 285
   Asn Thr Ala Thr Phe Glu Gly Met Tyr Leu Gly Thr Cys Gln Thr Leu
       290                 295                 300
   Thr Pro Leu Met Ser Ser Gln Phe Pro Glu Leu Gly Met Asn Pro Tyr
   305                 310                 315                 320
   His Cys Asn Glu Met Pro Trp Ile Lys Ser Ile Pro Phe Ile His Leu
                   325                 330                 335
   Gly Lys Glu Ala Ser Leu Val Asp Leu Leu Asn Arg Asn Asn Thr Phe
               340                 345                 350
   Lys Pro Phe Ala Glu Tyr Lys Ser Asp Tyr Val Tyr Gln Pro Phe Pro
           355                 360                 365
   Lys Pro Val Trp Glu Gln Ile Phe Gly Trp Leu Thr Lys Pro Gly Gly
       370                 375                 380
   Gly Met Met Ile Met Asp Pro Tyr Gly Ala Thr Ile Ser Ala Thr Pro
   385                 390                 395                 400
   Glu Ala Ala Thr Pro Phe Pro His Arg Gln Gly Val Leu Phe Asn Ile
                   405                 410                 415
   Gln Tyr Val Asn Tyr Trp Phe Ala Glu Ala Ala Ala Ala Ala Pro Leu
               420                 425                 430
   Gln Trp Ser Lys Asp Met Tyr Asn Phe Met Glu Pro Tyr Val Ser Lys
           435                 440                 445
   Asn Pro Arg Gln Ala Tyr Ala Asn Tyr Arg Asp Ile Asp Leu Gly Arg
       450                 455                 460
   Asn Glu Val Val Asn Asp Ile Ser Thr Tyr Ser Ser Gly Lys Val Trp
   465                 470                 475                 480
   Gly Glu Lys Tyr Phe Lys Gly Asn Phe Gln Arg Leu Ala Ile Thr Lys
                   485                 490                 495
   Gly Lys Val Asp Pro Gln Asp Tyr Phe Arg Asn Glu Gln Ser Ile Pro
               500                 505                 510
   Pro Leu Leu Glu Lys Tyr
           515
<210> 9
   <211> 1603
   <212> DNA
   <213> Triticum aestivum
<220>
   <221> stop_codon
   <222> (1555)..(1557)
   <223>
<220>
   <221> CDS
   <222> (1)..(1557)
   <223>
<220>
   <221> signal_sequence_DNA
   <222> (1)..(63)
   <223>
<220>
   <221> signal_sequence_PROT
   <222> (1)..(21)
   <223>
<400> 9
   aac tgt agg gcc ttc gcg cag gtg ctc ctc ttc ttc gcc ttg tcc tgc        48
   Asn Cys Arg Ala Phe Ala Gln Val Leu Leu Phe Phe Ala Leu Ser Cys
   1               5                   10                  15
   caa gcc gcc gcc acc tac gcg ccg gtg cct gcc aag gag gac ttc ctc        96
   Gln Ala Ala Ala Thr Tyr Ala Pro Val Pro Ala Lys Glu Asp Phe Leu
               20                  25                  30
   gga tgc ctc atg aag gag ata ccg gcc cgc ctc ctc tac gcc aag agc        144
   Gly Cys Leu Met Lys Glu Ile Pro Ala Arg Leu Leu Tyr Ala Lys Ser
           35                  40                  45
   tcg cct gac tac ccc acc gtg ctg gcg cag acc atc agg aac tcg cgg        192
   Ser Pro Asp Tyr Pro Thr Val Leu Ala Gln Thr Ile Arg Asn Ser Arg
       50                  55                  60
   tgg tcg acg cag cag aac gtg aag ccg ctg tac atc atc acc ccc acc        240
   Trp Ser Thr Gln Gln Asn Val Lys Pro Leu Tyr Ile Ile Thr Pro Thr
   65                  70                  75                  80
   aac gcc tcc cac atc caa tcc gcg gtg gtg tgc ggc cgc cgg cac ggc      288
   Asn Ala Ser His Ile Gln Ser Ala Val Val Cys Gly Arg Arg His Gly
                   85                  90                  95
   gtc cgc ctc cgc gtg cgg agc ggc ggc cac gac tac gag ggc ctg tcg      336
   Val Arg Leu Arg Val Arg Ser Gly Gly His Asp Tyr Glu Gly Leu Ser
               100                 105                 110
   tac cgg tcc gag aaa ccc gag acg ttc gcc gtc gtc gac ctc aac aag      384
   Tyr Arg Ser Glu Lys Pro Glu Thr Phe Ala Val Val Asp Leu Asn Lys
           115                 120                 125
   atg cgg gca gtg gtt gtc gac ggc tac gcc cgc acg gcg tgg gtc gaa      432
   Met Arg Ala Val Val Val Asp Gly Tyr Ala Arg Thr Ala Trp Val Glu
       130                 135                 140
   tcc ggc gcg cag ctc ggc gag ctc tac tac gcc atc gcg aag aac agc      480
   Ser Gly Ala Gln Leu Gly Glu Leu Tyr Tyr Ala Ile Ala Lys Asn Ser
   145                 150                 155                 160
   ccc gtg ctc gcg ttc ccg gcc ggc gtc tgc ccg tcc atc ggc gtc ggc      528
   Pro Val Leu Ala Phe Pro Ala Gly Val Cys Pro Ser Ile Gly Val Gly
                   165                 170                 175
   ggc aac ttc gca ggc ggc ggc ttc ggc atg ctg ctg cgc aag tac ggc      576
   Gly Asn Phe Ala Gly Gly Gly Phe Gly Met Leu Leu Arg Lys Tyr Gly
               180                 185                 190
   atc gcc gcc gag aac gtc atc gac gtc aag gtg gtc gac ccc gac ggc      624
   Ile Ala Ala Glu Asn Val Ile Asp Val Lys Val Val Asp Pro Asp Gly
           195                 200                 205
   aag ctg ctc gac aag agc tcc atg agc gcg gac cac ttc tgg gcc gtc      672
   Lys Leu Leu Asp Lys Ser Ser Met Ser Ala Asp His Phe Trp Ala Val
       210                 215                 220
   agg ggc ggc ggc gga gag agc ttc ggc atc gtc gtc tcg tgg cag gtg      720
   Arg Gly Gly Gly Gly Glu Ser Phe Gly Ile Val Val Ser Trp Gln Val
   225                 230                 235                 240
   aag ctc atg cca gtg cct ccc acc gtc acc gtg ttt aag atc ccc aag      768
   Lys Leu Met Pro Val Pro Pro Thr Val Thr Val Phe Lys Ile Pro Lys
                   245                 250                 255
   acg gtg caa gaa ggc gcc gta gac ctc gtc aac aag tgg cag ctg gtc      816
   Thr Val Gln Glu Gly Ala Val Asp Leu Val Asn Lys Trp Gln Leu Val
               260                 265                 270
   ggg ccg gca ctt ccc ggc gac ctc atg atc cgc gtc atc gct gcc ggg      864
   Gly Pro Ala Leu Pro Gly Asp Leu Met Ile Arg Val Ile Ala Ala Gly
           275                 280                 285
   aac acg gcg acg ttc gag gcc ttg tac ctg ggc acc tgc aaa acc ctg      912
   Asn Thr Ala Thr Phe Glu Ala Leu Tyr Leu Gly Thr Cys Lys Thr Leu
       290                 295                 300
   acg ccg ctg atg agc agc caa ttc ccc gag ctt ggc atg aac ccc tat      960
   Thr Pro Leu Met Ser Ser Gln Phe Pro Glu Leu Gly Met Asn Pro Tyr
   305                 310                 315                 320
   cac tgc aac gag atg ccc tgg atc aag tcc gtc ccc ttc atc cac ctc     1008
   His Cys Asn Glu Met Pro Trp Ile Lys Ser Val Pro Phe Ile His Leu
                   325                 330                 335
   ggc aaa cag gct ggc ctg gac gac ctc ctc aac cgg aac aac acc ttc      1056
   Gly Lys Gln Ala Gly Leu Asp Asp Leu Leu Asn Arg Asn Asn Thr Phe
               340                 345                 350
   aag ccc ttc gcc gaa tac aag tcg gac tac gtg tac cag ccc ttc ccc      1104
   Lys Pro Phe Ala Glu Tyr Lys Ser Asp Tyr Val Tyr Gln Pro Phe Pro
           355                 360                 365
   aag ccc gtg tgg gag cag atc ttc ggc tgg ctc gcg aag ccc ggc gcg      1152
   Lys Pro Val Trp Glu Gln Ile Phe Gly Trp Leu Ala Lys Pro Gly Ala
       370                 375                 380
   ggg atc atg atc atg gac ccc tac ggc gcc acc atc agc gcc acc ccc      1200
   Gly Ile Met Ile Met Asp Pro Tyr Gly Ala Thr Ile Ser Ala Thr Pro
   385                 390                 395                 400
   gaa gcg gcg acg ccg ttc cct cac cgc cag ggc gtc ctc ttc aac atc      1248
   Glu Ala Ala Thr Pro Phe Pro His Arg Gln Gly Val Leu Phe Asn Ile
                   405                 410                 415
   cag tat gtc aac tac tgg ttc gcc gag cca gcc ggc gcc gcg ccg ctg      1296
   Gln Tyr Val Asn Tyr Trp Phe Ala Glu Pro Ala Gly Ala Ala Pro Leu
               420                 425                 430
   cag tgg agc aag gac att tac aat ttc atg gag ccg tac gtg agc aag      1344
   Gln Trp Ser Lys Asp Ile Tyr Asn Phe Met Glu Pro Tyr Val Ser Lys
           435                 440                 445
   aac ccc agg cag gcg tac gcc aac tac agg gac atc gac ctc ggc agg      1392
   Asn Pro Arg Gln Ala Tyr Ala Asn Tyr Arg Asp Ile Asp Leu Gly Arg
       450                 455                 460
   aat gag gtg gtg aac gac atc tca acc tac agc agc ggc aag gtg tgg      1440
   Asn Glu Val Val Asn Asp Ile Ser Thr Tyr Ser Ser Gly Lys Val Trp
   465                 470                 475                 480
   ggc gag aag tac ttc aag agc aac ttc caa agg ctc gcc att acc aag      1488
   Gly Glu Lys Tyr Phe Lys Ser Asn Phe Gln Arg Leu Ala Ile Thr Lys
                   485                 490                 495
   ggc aag gta gat cct cag gac tac ttc agg aat gag caa agc atc ccg      1536
   Gly Lys Val Asp Pro Gln Asp Tyr Phe Arg Asn Glu Gln Ser Ile Pro
               500                 505                 510
   ccg ctg atc gag aag tac tga tcgaggacct tgcatggaga tttagtgcgt        1587
   Pro Leu Ile Glu Lys Tyr
           515
   ggttggcgtt tcacat                                                    1603
<210> 10
   <211> 518
   <212> PRT
   <213> Triticum aestivum
<400> 10
   Asn Cys Arg Ala Phe Ala Gln Val Leu Leu Phe Phe Ala Leu Ser Cys
   1               5                   10                  15
   Gln Ala Ala Ala Thr Tyr Ala Pro Val Pro Ala Lys Glu Asp Phe Leu
               20                  25                  30
   Gly Cys Leu Met Lys Glu Ile Pro Ala Arg Leu Leu Tyr Ala Lys Ser
           35                  40                  45
   Ser Pro Asp Tyr Pro Thr Val Leu Ala Gln Thr Ile Arg Asn Ser Arg
       50                  55                  60
   Trp Ser Thr Gln Gln Asn Val Lys Pro Leu Tyr Ile Ile Thr Pro Thr
   65                  70                  75                  80
   Asn Ala Ser His Ile Gln Ser Ala Val Val Cys Gly Arg Arg His Gly
                   85                  90                  95
   Val Arg Leu Arg Val Arg Ser Gly Gly His Asp Tyr Glu Gly Leu Ser
               100                 105                 110
   Tyr Arg Ser Glu Lys Pro Glu Thr Phe Ala Val Val Asp Leu Asn Lys
           115                 120                 125
   Met Arg Ala Val Val Val Asp Gly Tyr Ala Arg Thr Ala Trp Val Glu
       130                 135                 140
   Ser Gly Ala Gln Leu Gly Glu Leu Tyr Tyr Ala Ile Ala Lys Asn Ser
   145                 150                 155                 160
   Pro Val Leu Ala Phe Pro Ala Gly Val Cys Pro Ser Ile Gly Val Gly
                   165                 170                 175
   Gly Asn Phe Ala Gly Gly Gly Phe Gly Met Leu Leu Arg Lys Tyr Gly
               180                 185                 190
   Ile Ala Ala Glu Asn Val Ile Asp Val Lys Val Val Asp Pro Asp Gly
           195                 200                 205
   Lys Leu Leu Asp Lys Ser Ser Met Ser Ala Asp His Phe Trp Ala Val
       210                 215                 220
   Arg Gly Gly Gly Gly Glu Ser Phe Gly Ile Val Val Ser Trp Gln Val
   225                 230                 235                 240
   Lys Leu Met Pro Val Pro Pro Thr Val Thr Val Phe Lys Ile Pro Lys
                   245                 250                 255
   Thr Val Gln Glu Gly Ala Val Asp Leu Val Asn Lys Trp Gln Leu Val
               260                 265                 270
   Gly Pro Ala Leu Pro Gly Asp Leu Met Ile Arg Val Ile Ala Ala Gly
           275                 280                 285
   Asn Thr Ala Thr Phe Glu Ala Leu Tyr Leu Gly Thr Cys Lys Thr Leu
       290                 295                 300
   Thr Pro Leu Met Ser Ser Gln Phe Pro Glu Leu Gly Met Asn Pro Tyr
   305                 310                 315                 320
   His Cys Asn Glu Met Pro Trp Ile Lys Ser Val Pro Phe Ile His Leu
                   325                 330                 335
   Gly Lys Gln Ala Gly Leu Asp Asp Leu Leu Asn Arg Asn Asn Thr Phe
               340                 345                 350
   Lys Pro Phe Ala Glu Tyr Lys Ser Asp Tyr Val Tyr Gln Pro Phe Pro
           355                 360                 365
   Lys Pro Val Trp Glu Gln Ile Phe Gly Trp Leu Ala Lys Pro Gly Ala
       370                 375                 380
   Gly Ile Met Ile Met Asp Pro Tyr Gly Ala Thr Ile Ser Ala Thr Pro
   385                 390                 395                 400
   Glu Ala Ala Thr Pro Phe Pro His Arg Gln Gly Val Leu Phe Asn Ile
                   405                 410                 415
   Gln Tyr Val Asn Tyr Trp Phe Ala Glu Pro Ala Gly Ala Ala Pro Leu
               420                 425                 430
   Gln Trp Ser Lys Asp Ile Tyr Asn Phe Met Glu Pro Tyr Val Ser Lys
           435                 440                 445
   Asn Pro Arg Gln Ala Tyr Ala Asn Tyr Arg Asp Ile Asp Leu Gly Arg
       450                 455                 460
   Asn Glu Val Val Asn Asp Ile Ser Thr Tyr Ser Ser Gly Lys Val Trp
   465                 470                 475                 480
   Gly Glu Lys Tyr Phe Lys Ser Asn Phe Gln Arg Leu Ala Ile Thr Lys
                   485                 490                 495
   Gly Lys Val Asp Pro Gln Asp Tyr Phe Arg Asn Glu Gln Ser Ile Pro
               500                 505                 510
   Pro Leu Ile Glu Lys Tyr
           515
<210> 11
   <211> 1503
   <212> DNA
   <213> Phleum pratense
<220>
   <221> CDS
   <222> (1)..(1503)
   <223>
<400> 11
   tac ttc ccg ccg ccg gct gct aaa gaa gac ttc ctg ggt tgc ctg gtt      48
   Tyr Phe Pro Pro Pro Ala Ala Lys Glu Asp Phe Leu Gly Cys Leu Val
   1               5                   10                  15
   aaa gaa atc ccg ccg cgt ctg ttg tac gcg aaa tcg tcg ccg gcg tat      96
   Lys Glu Ile Pro Pro Arg Leu Leu Tyr Ala Lys Ser Ser Pro Ala Tyr
               20                  25                  30
   ccc tca gtc ctg ggg cag acc atc cgg aac tcg agg tgg tcg tcg ccg     144
   Pro Ser Val Leu Gly Gln Thr Ile Arg Asn Ser Arg Trp Ser Ser Pro
           35                  40                  45
   gac aac gtg aag ccg ctc tac atc atc acc ccc acc aac gtc tcc cac     192
   Asp Asn Val Lys Pro Leu Tyr Ile Ile Thr Pro Thr Asn Val Ser His
       50                  55                  60
   atc cag tcc gcc gtg gtg tgc ggc cgc cgc cac agc gtc cgc atc cgc     240
   Ile Gln Ser Ala Val Val Cys Gly Arg Arg His Ser Val Arg Ile Arg
   65                  70                  75                  80
   gtg cgc agc ggc ggg cac gac tac gag ggc ctc tcg tac cgg tct ttg     288
   Val Arg Ser Gly Gly His Asp Tyr Glu Gly Leu Ser Tyr Arg Ser Leu
                   85                  90                  95
   cag ccc gag acg ttc gcc gtc gtc gac ctc aac aag atg cgg gcg gtg     336
   Gln Pro Glu Thr Phe Ala Val Val Asp Leu Asn Lys Met Arg Ala Val
               100                 105                 110
   tgg gtg gac ggc aag gcc cgc acg gcg tgg gtg gac tcc ggc gcg cag     384
   Trp Val Asp Gly Lys Ala Arg Thr Ala Trp Val Asp Ser Gly Ala Gln
           115                 120                 125
   ctc ggc gag ctc tac tac gcc atc tat aag gcg agc ccc acg ctg gcg       432
   Leu Gly Glu Leu Tyr Tyr Ala Ile Tyr Lys Ala Ser Pro Thr Leu Ala
       130                 135                 140
   ttc ccg gcc ggc gtg tgc ccg acg atc gga gtg ggc ggc aac ttc gcg       480
   Phe Pro Ala Gly Val Cys Pro Thr Ile Gly Val Gly Gly Asn Phe Ala
   145                 150                 155                 160
   ggc ggc ggc ttc ggc atg ctg ctg cgc aag tac ggc atc gcc gcg gag       528
   Gly Gly Gly Phe Gly Met Leu Leu Arg Lys Tyr Gly Ile Ala Ala Glu
                   165                 170                 175
   aac gtc atc gac gtg aag ctc gtc gac gcc aac ggc aag ctg cac gac       576
   Asn Val Ile Asp Val Lys Leu Val Asp Ala Asn Gly Lys Leu His Asp
               180                 185                 190
   aag aag tcc atg ggc gac gac cat ttc tgg gcc gtc agg ggc ggc ggg       624
   Lys Lys Ser Met Gly Asp Asp His Phe Trp Ala Val Arg Gly Gly Gly
           195                 200                 205
   ggc gag agc ttc ggc atc gtg gtc gcg tgg cag gtg aag ctc ctg ccg       672
   Gly Glu Ser Phe Gly Ile Val Val Ala Trp Gln Val Lys Leu Leu Pro
       210                 215                 220
   gtg ccg ccc acc gtg aca ata ttc aag atc tcc aag aca gtg agc gag       720
   Val Pro Pro Thr Val Thr Ile Phe Lys Ile Ser Lys Thr Val Ser Glu
   225                 230                 235                 240
   ggc gcc gtg gac atc atc aac aag tgg caa gtg gtc gcg ccg cag ctt       768
   Gly Ala Val Asp Ile Ile Asn Lys Trp Gln Val Val Ala Pro Gln Leu
                   245                 250                 255
   ccc gcc gac ctc atg atc cgc atc atc gcg cag ggg ccc aag gcc acg       816
   Pro Ala Asp Leu Met Ile Arg Ile Ile Ala Gln Gly Pro Lys Ala Thr
               260                 265                 270
   ttc gag gcc atg tac ctc ggc acc tgc aaa acc ctg acg ccg ttg atg       864
   Phe Glu Ala Met Tyr Leu Gly Thr Cys Lys Thr Leu Thr Pro Leu Met
           275                 280                 285
   agc agc aag ttc ccg gag ctc ggc atg aac ccc tcc cac tgc aac gag       912
   Ser Ser Lys Phe Pro Glu Leu Gly Met Asn Pro Ser His Cys Asn Glu
       290                 295                 300
   atg tca tgg atc cag tcc atc ccc ttc gtc cac ctc ggc cac agg gac       960
   Met Ser Trp Ile Gln Ser Ile Pro Phe Val His Leu Gly His Arg Asp
   305                 310                 315                 320
   gcc ctc gag gac gac ctc ctc aac cgg aac aac tcc ttc aag ccc ttc      1008
   Ala Leu Glu Asp Asp Leu Leu Asn Arg Asn Asn Ser Phe Lys Pro Phe
                   325                 330                 335
   gcc gaa tac aag tcc gac tac gtc tac cag ccc ttc ccc aag acc gtc      1056
   Ala Glu Tyr Lys Ser Asp Tyr Val Tyr Gln Pro Phe Pro Lys Thr Val
               340                 345                 350
   tgg gag cag atc ctc aac acc tgg ctc gtc aag ccc ggc gcc ggg atc      1104
   Trp Glu Gln Ile Leu Asn Thr Trp Leu Val Lys Pro Gly Ala Gly Ile
           355                 360                 365
   atg atc ttc gac ccc tac ggc gcc acc atc agc gcc acc ccg gag tcc      1152
   Met Ile Phe Asp Pro Tyr Gly Ala Thr Ile Ser Ala Thr Pro Glu Ser
       370                 375                 380
   gcc acg ccc ttc cct cac cgc aag ggc gtc ctc ttc aac atc cag tac      1200
   Ala Thr Pro Phe Pro His Arg Lys Gly Val Leu Phe Asn Ile Gln Tyr
   385                 390                 395                 400
   gtc aac tac tgg ttc gcc ccg gga gcc gcc gcc gcg ccc ctc tcg tgg      1248
   Val Asn Tyr Trp Phe Ala Pro Gly Ala Ala Ala Ala Pro Leu Ser Trp
                   405                 410                 415
   agc aag gac atc tac aac tac atg gag ccc tac gtg agc aag aac ccc      1296
   Ser Lys Asp Ile Tyr Asn Tyr Met Glu Pro Tyr Val Ser Lys Asn Pro
               420                 425                 430
   agg cag gcg tac gca aac tac agg gac atc gac ctc ggc agg aac gag      1344
   Arg Gln Ala Tyr Ala Asn Tyr Arg Asp Ile Asp Leu Gly Arg Asn Glu
           435                 440                 445
   gtg gtc aac gac gtc tcc acc tac gcc agc ggc aag gtc tgg ggc cag      1392
   Val Val Asn Asp Val Ser Thr Tyr Ala Ser Gly Lys Val Trp Gly Gln
       450                 455                 460
   aaa tac ttc aag ggc aac ttc gag agg ctc gcc att acc aag ggc aag      1440
   Lys Tyr Phe Lys Gly Asn Phe Glu Arg Leu Ala Ile Thr Lys Gly Lys
   465                 470                 475                 480
   gtc gat cct acc gac tac ttc agg aac gag cag agc atc ccg ccg ctc      1488
   Val Asp Pro Thr Asp Tyr Phe Arg Asn Glu Gln Ser Ile Pro Pro Leu
                   485                 490                 495
   atc aaa aag tac tga                                                  1503
   Ile Lys Lys Tyr
               500
<210> 12
   <211> 500
   <212> PRT
   <213> Phleum pratense
<400> 12
   Tyr Phe Pro Pro Pro Ala Ala Lys Glu Asp Phe Leu Gly Cys Leu Val
   1               5                   10                  15
   Lys Glu Ile Pro Pro Arg Leu Leu Tyr Ala Lys Ser Ser Pro Ala Tyr
               20                  25                  30
   Pro Ser Val Leu Gly Gln Thr Ile Arg Asn Ser Arg Trp Ser Ser Pro
           35                  40                  45
   Asp Asn Val Lys Pro Leu Tyr Ile Ile Thr Pro Thr Asn Val Ser His
       50                  55                  60
   Ile Gln Ser Ala Val Val Cys Gly Arg Arg His Ser Val Arg Ile Arg
   65                  70                  75                  80
   Val Arg Ser Gly Gly His Asp Tyr Glu Gly Leu Ser Tyr Arg Ser Leu
                   85                  90                  95
   Gln Pro Glu Thr Phe Ala Val Val Asp Leu Asn Lys Met Arg Ala Val
               100                 105                 110
   Trp Val Asp Gly Lys Ala Arg Thr Ala Trp Val Asp Ser Gly Ala Gln
           115                 120                 125
   Leu Gly Glu Leu Tyr Tyr Ala Ile Tyr Lys Ala Ser Pro Thr Leu Ala
       130                 135                 140
   Phe Pro Ala Gly Val Cys Pro Thr Ile Gly Val Gly Gly Asn Phe Ala
   145                 150                 155                 160
   Gly Gly Gly Phe Gly Met Leu Leu Arg Lys Tyr Gly Ile Ala Ala Glu
                   165                 170                 175
   Asn Val Ile Asp Val Lys Leu Val Asp Ala Asn Gly Lys Leu His Asp
               180                 185                 190
   Lys Lys Ser Met Gly Asp Asp His Phe Trp Ala Val Arg Gly Gly Gly
           195                 200                 205
   Gly Glu Ser Phe Gly Ile Val Val Ala Trp Gln Val Lys Leu Leu Pro
       210                 215                 220
   Val Pro Pro Thr Val Thr Ile Phe Lys Ile Ser Lys Thr Val Ser Glu
   225                 230                 235                 240
   Gly Ala Val Asp Ile Ile Asn Lys Trp Gln Val Val Ala Pro Gln Leu
                   245                 250                 255
   Pro Ala Asp Leu Met Ile Arg Ile Ile Ala Gln Gly Pro Lys Ala Thr
               260                 265                 270
   Phe Glu Ala Met Tyr Leu Gly Thr Cys Lys Thr Leu Thr Pro Leu Met
           275                 280                 285
   Ser Ser Lys Phe Pro Glu Leu Gly Met Asn Pro Ser His Cys Asn Glu
       290                 295                 300
   Met Ser Trp Ile Gln Ser Ile Pro Phe Val His Leu Gly His Arg Asp
   305 310 315 320
   Ala Leu Glu Asp Asp Leu Leu Asn Arg Asn Asn Ser Phe Lys Pro Phe
   325 330 335
   Ala Glu Tyr Lys Ser Asp Tyr Val Tyr Gln Pro Phe Pro Lys Thr Val
   340 345 350
   Trp Glu Gln Ile Leu Asn Thr Trp Leu Val Lys Pro Gly Ala Gly Ile
   355 360 365
   Met Ile Phe Asp Pro Tyr Gly Ala Thr Ile Ser Ala Thr Pro Glu Ser
   370 375 380
   Ala Thr Pro Phe Pro His Arg Lys Gly Val Leu Phe Asn Ile Gln Tyr
   385 390 395 400
   Val Asn Tyr Trp Phe Ala Pro Gly Ala Ala Ala Ala Pro Leu Ser Trp
   405 410 415
   Ser Lys Asp Ile Tyr Asn Tyr Met Glu Pro Tyr Val Ser Lys Asn Pro
   420 425 430
   Arg Gln Ala Tyr Ala Asn Tyr Arg Asp Ile Asp Leu Gly Arg Asn Glu
   435 440 445
   Val Val Asn Asp Val Ser Thr Tyr Ala Ser Gly Lys Val Trp Gly Gln
   450 455 460
   Lys Tyr Phe Lys Gly Asn Phe Glu Arg Leu Ala Ile Thr Lys Gly Lys
   465 470 475 480
   Val Asp Pro Thr Asp Tyr Phe Arg Asn Glu Gln Ser Ile Pro Pro Leu
   485 490 495
   Ile Lys Lys Tyr
   500
<210> 13
   <211> 12
   <212> PRT
   <213> Dactylus glomerata
<400> 13
   Asp Ile Tyr Asn Tyr Met Glu Pro Tyr Val Ser Lys
   1 5 10
<210> 14
   <211> 11
   <212> PRT
   <213> Dactylus glomerata
<400> 14
   Val Asp Pro Thr Asp Tyr Phe Gly Asn Glu Gln
   1               5                   10
<210> 15
   <211> 17
   <212> PRT
   <213> Dactylus glomerata
<400> 15
   Ala Arg Thr Ala Trp Val Asp Ser Gly Ala Gln Leu Gly Glu Leu Ser
   1               5                   10                  15
   Tyr
<210> 16
   <211> 15
   <212> PRT
   <213> Dactylus glomerata
<400> 16
   Gly Val Leu Phe Asn Ile Gln Tyr Val Asn Tyr Trp Phe Ala Pro
   1               5                   10                  15
<210> 17
   <211> 11
   <212> PRT
   <213> Cynodon dactylon
<400> 17
   Lys Thr Val Lys Pro Leu Tyr Ile Ile Thr Pro
   1               5                   10
<210> 18
   <211> 22
   <212> PRT
   <213> Cynodon dactylon
<400> 18
   Lys Gln Val Glu Arg Asp Phe Leu Thr Ser Leu Thr Lys Asp Ile Pro
   1               5                   10                  15
   Gln Leu Tyr Leu Lys Ser
               20
<210> 19
   <211> 16
   <212> PRT
   <213> Cynodon dactylon
<400> 19
   Thr Val Lys Pro Leu Tyr Ile Ile Thr Pro Ile Thr Ala Ala Met Ile
   1 5 10 15
<210> 20
   <211> 24
   <212> PRT
   <213> Cynodon dactylon
<400> 20
   Leu Arg Lys Tyr Gly Thr Ala Ala Asp Asn Val Ile Asp Ala Lys Val
   1 5 10 15
   Val Asp Ala Gln Gly Arg Leu Leu
   20
<210> 21
   <211> 14
   <212> PRT
   <213> Cynodon dactylon
<400> 21
   Lys Trp Gln Thr Val Ala Pro Ala Leu Pro Asp Pro Asn Met
   1 5 10
<210> 22
   <211> 15
   <212> PRT
   <213> Cynodon dactylon
<400> 22
   Val Thr Trp Ile Glu Ser Val Pro Tyr Ile Pro Met Gly Asp Lys
   1 5 10 15
<210> 23
   <211> 19
   <212> PRT
   <213> Cynodon dactylon
<220>
   <221> MISC_FEATURE
   <222> (8)..(8)
   <223> undetermined amino acid
<400> 23
   Gly Thr Val Arg Asp Leu Leu Xaa Arg Thr Ser Asn Ile Lys Ala Phe
   1 5 10 15
   Gly Lys Tyr
<210> 24
   <211> 23
   <212> PRT
   <213> Cynodon dactylon
<400> 24
   Thr Ser Asn Ile Lys Ala Phe Gly Lys Tyr Lys Ser Asp Tyr Val Leu
   1               5                   10                  15
   Glu Pro Ile Pro Lys Lys Ser
               20
<210> 25
   <211> 13
   <212> PRT
   <213> Cynodon dactylon
<400> 25
   Tyr Arg Asp Leu Asp Leu Gly Val Asn Gln Val Val Gly
   1               5                   10
<210> 26
   <211> 15
   <212> PRT
   <213> Cynodon dactylon
<400> 26
   Ser Ala Thr Pro Pro Thr His Arg Ser Gly Val Leu Phe Asn Ile
   1               5                   10                  15
<210> 27
   <211> 36
   <212> PRT
   <213> Cynodon dactylon
<400> 27
   Ala Ala Ala Ala Leu Pro Thr Gln Val Thr Arg Asp Ile Tyr Ala Phe
   1               5                   10                  15
   Met Thr Pro Tyr Val Ser Lys Asn Pro Arg Gln Ala Tyr Val Asn Tyr
               20                  25                  30
   Arg Asp Leu Asp
           35
<210> 28
   <211> 14
   <212> PRT
   <213> Lolium perenne
<400> 28
   Phe Leu Glu Pro Val Leu Gly Leu Ile Phe Pro Ala Gly Val
   1 5 10
<210> 29
   <211> 9
   <212> PRT
   <213> Lolium perenne
<400> 29
   Gly Leu Ile Glu Phe Pro Ala Gly Val
   1 5
<210> 30
   <211> 22
   <212> DNA
   <213> Secale cereale
<400> 30
   ggctcccggg gcgaaccagt ag 22
<210> 31
   <211> 23
   <212> DNA
   <213> Secale cereale
<400> 31
   accaacgcct cccacatcca gtc 23
<210> 32
   <211> 49
   <212> DNA
   <213> Secale cereale
<400> 32
   gataagcttc tcgagtgatt agtacttttt gatcagcggc gggatgctc 49
<210> 33
   <211> 23
   <212> DNA
   <213> Secale cereale
<400> 33
   gctctcgatc ggctacaatg gcg 23
<210> 34
   <211> 25
   <212> DNA
   <213> Secale cereale
<400> 34
   cacgcactac aaatctccat gcaag 25
<210> 35
   <211> 30
   <212> DNA
   <213> Secale cereale
<400> 35
   catgcttgat ccttattcta ctagttgggc 30
<210> 36
   <211> 30
   <212> DNA
   <213> Secale cereale
<400> 36
   tacgcacgat ccttattcta ctagttgggc 30
<210> 37
   <211> 30
   <212> DNA
   <213> Hordeum vulgare
<400> 37
   gccttgtcct gccaccacgc cgccgccacc 30
<210> 38
   <211> 23
   <212> DNA
   <213> Hordeum vulgare
<400> 38
   gctctcgatc ggctacaatg gcg 23
<210> 39
   <211> 25
   <212> DNA
   <213> Hordeum vulgare
<400> 39
   cacgcactac aaatctccat gcaag 25
<210> 40
   <211> 30
   <212> DNA
   <213> Hordeum vulgare
<400> 40
   catgcttgat ccttattcta ctagttgggc 30
<210> 41
   <211> 23
   <212> DNA
   <213> Triticum aestivum
<400> 41
   cacgcactaa atctccatgc aag 23
<210> 42
   <211> 30
   <212> DNA
   <213> Triticum aestivum
<400> 42
   tacgcacgat ccttattcta ctagttgggc 30
<210> 43
   <211> 23
   <212> DNA
   <213> Triticum aestivum
<400> 43
   aagctctatc gcctacaatg gcg 23
<210> 44
   <211> 25
   <212> DNA
   <213> Triticum aestivum
<400> 44
   ggtgctcctc ttctgcgcct tgtcc 25